# EUROPEAN PATENT SPECIFICATION

(11) **EP 3 201 353 B1**
(45) Date of publication and mention of the grant of the patent: **25.12.2019**
(21) Application number: 15777645.1
(22) Date of filing: 30.09.2015
(51) Int. Cl.: C12Q 1/6811, C12N 15/10, C12N 15/11

(54) **A METHOD OF IDENTIFYING OR PRODUCING AN APTAMER**
VERFAHREN ZUR IDENTIFIZIERUNG ODER HERSTELLUNG EINES APTAMERS
PROCÉDÉ D'IDENTIFICATION OU DE PRODUCTION D'UN APTAMÈRE

(30) Priority: 02.10.2014 US 201462058703 P
(43) Date of publication of application: 09.08.2017
(73) Proprietor: Rheinische Friedrich-Wilhelms-Universität Bonn, 53113 Bonn (DE)
(72) Inventor: MAYER, Guenter, 53123 Bonn (DE); TOLLE, Fabian, CB2 9AA, Cambridge (GB)
(74) Representative: Michalski Hüttermann & Partner Patentanwälte mbB
(86) International application number: PCT/EP2015/072566
(87) International publication number: WO 2016/050850

(56) References cited:
- WO-A1-2009/012410
- WO-A1-2015/049356
- US-A1- 2011 210 017
- US-A1- 2012 115 752
- US-A1- 2014 100 120
- YUNFENG CHENG ET AL: "Design, Synthesis, and Polymerase-Catalyzed Incorporation of Click-Modified Boronic Acid-TTP Analogues", CHEMISTRY - AN ASIAN JOURNAL, vol. 6, no. 10, 4 October 2011 (2011-10-04), pages 2747-2752, XP055160640, ISSN: 1861-4728, DOI: 10.1002/asia.201100229
- SAMRA OBEID ET AL: "Interactions of non-polar and "Click-able" nucleotides in the confines of a DNA polymerase active site", CHEMICAL COMMUNICATIONS, vol. 48, no. 67, 1 January 2012 (2012-01-01), page 8320, XP055160951, ISSN: 1359-7345, DOI: 10.1039/c2cc34181f
- GRAMLICH PHILIPP M E ET AL: "Postsynthetic DNA modification through the copper-catalyzed azide-alkyne cycloaddition reaction", ANGEWANDTE CHEMIE INTERNATIONAL EDITION, WILEY - V C H VERLAG GMBH & CO. KGAA, DE, vol. 47, no. 44, 20 October 2008 (2008-10-20), pages 8350-8358, XP009120328, ISSN: 1433-7851, DOI: 10.1002/ANIE.200802077 [retrieved on 2008-09-22]
- PHILIPP M E GRAMLICH ET AL: "Click?Click?Click: Single to Triple Modification of DNA", ANGEWANDTE CHEMIE INTERNATIONAL EDITION, WILEY - V C H VERLAG GMBH & CO. KGAA, DE, vol. 47, no. 18, 1 January 2008 (2008-01-01), pages 3442-3444, XP007916867, ISSN: 1433-7851, DOI: 10.1002/ANIE.200705664 [retrieved on 2008-03-28]
- KEEFE A D ET AL: "SELEX with modified nucleotides", CURRENT OPINION IN CHEMICAL BIOLOGY, CURRENT BIOLOGY LTD, LONDON, GB, vol. 12, no. 4, 1 August 2008 (2008-08-01) , pages 448-456, XP024528065, ISSN: 1367-5931, DOI: 10.1016/J.CBPA.2008.06.028 [retrieved on 2008-07-21]
- MAYER G ET AL: "From selection to caged aptamers: Identification of light-dependent ssDNA aptamers targeting cytohesin", BIOORGANIC & MEDICINAL CHEMISTRY LETTERS, PERGAMON, AMSTERDAM, NL, vol. 19, no. 23, 1 December 2009 (2009-12-01), pages 6561-6564, XP026736055, ISSN: 0960-894X, DOI: 10.1016/J.BMCL.2009.10.032 [retrieved on 2009-10-14]
- CLARA BRIEKE ET AL: "Light-Controlled Tools", ANGEWANDTE CHEMIE INTERNATIONAL EDITION, vol. 51, no. 34, 20 August 2012 (2012-08-20), pages 8446-8476, XP055160738, ISSN: 1433-7851, DOI: 10.1002/anie.201202134
- FABIAN TOLLE ET AL: "Dressed for success - applying chemistry to modulate aptamer functionality", CHEMICAL SCIENCE, vol. 4, no. 1, 1 January 2013 (2013-01-01) , page 60, XP055160479, ISSN: 2041-6520, DOI: 10.1039/c2sc21510a
- VARATHARASA THIVIYANATHAN ET AL: "Aptamers and the next generation of diagnostic reagents", PROTEOMICS - CLINICAL APPLICATIONS, vol. 6, no. 11-12, 15 December 2012 (2012-12-15), pages 563-573, XP055160488, ISSN: 1862-8346, DOI: 10.1002/prca.201200042
- LATHAM J A ET AL: "The application of a modified nucleotide in aptamer selection : novel thrombin aptamers containing 5-(1-pentynyl)-2'-deoxyuridine", NUCLEIC ACIDS RESEARCH, OXFORD UNIVERSITY PRESS, GB, vol. 22, no. 14, 1 January 1994 (1994-01-01), pages 2817-2822, XP002176163, ISSN: 0305-1048
- MOHAMMAD MEHEDI MASUD ET AL: "Sialyllactose-binding modified DNA aptamer bearing additional functionality by SELEX", BIOORGANIC & MEDICINAL CHEMISTRY, vol. 12, no. 5, 1 March 2004 (2004-03-01), pages 1111-1120, XP055160822, ISSN: 0968-0896, DOI: 10.1016/j.bmc.2003.12.009
- KOLB H C ET AL: "The growing impact of click chemistry on drug discovery", DRUG DISCOVERY TODAY, ELSEVIER, RAHWAY, NJ, US, vol. 8, no. 24, 15 December 2003 (2003-12-15), pages 1128-1137, XP002377521, ISSN: 1359-6446, DOI: 10.1016/S1359-6446(03)02933-7

## Description

### FIELD OF THE INVENTION

The present invention relates to the technical fields of techniques for producing oligonucleotides that specifically bind to a target. The present invention relates to a method of identifying or producing an aptamer.

### BACKGROUND OF THE INVENTION

Systematic Evolution of Ligands by Exponential Enrichment (SELEX), also referred to as in vitro selection or in vitro evolution, is a technique for producing oligonucleotides of either single-stranded DNA or RNA that specifically bind to a target ligand. The SELEX method is for example described in document US 5270163. In the SELEX method, a candidate mixture of single stranded nucleic acids having regions of randomized sequence is contacted with a target structure and those nucleic acids having an increased affinity to the target are selected and amplified. After several iterations a nucleic acid with good affinity to the target is obtained. Keefe and Cload (Current Opinion in Chemical Biology 2008, 12:448-456) disclose SELEX with modified nucleotides. US 2914/100120 discloses aptamers chemically synthesized to contain 5-ethynyl-dU.

Many targets are difficult to address using traditional SELEX approaches. One reason may be the limited chemical diversity of nucleic acids. Chemically modified nucleic acids add diversity that can address this issue. However, chemically modified nucleotide-triphosphates may lack compatibility with enzymatic steps, e.g. the polymerase chain reaction (PCR), of the in vitro selection process. Hence, the possible chemical modifications of DNA molecules suitable for in vitro selection are limited. From a practical point of view, use of such modifications is not easily to accomplish since most of them are not commercially accessible and, thus, require laborious chemical synthesis prior use.

Therefore, it is an object of the present invention to provide a method that allows for the introduction of additional chemical entities into nucleic acids during the aptamer selection process.

### SUMMARY OF THE INVENTION

The present invention is defined in appended claims 1 to 6.

It is provided a method of identifying or producing an aptamer comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the nucleobase that has been modified to contain the azide-alkyne group was an ethynyl-dU, dA, dC or dG nucleotide;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer,wherein the binding nucleic acids are amplified by PCR using an ethynyl-modified nucleobase and again are subjected to an in vitro selection cycle until an enrichment of target-binding nucleic acids is detected.

It has been surprisingly found that the method enables to introduce additional chemical entities into DNA during the selection process. Advantageously, the modification is only transiently present during the selection cycle. This enables a high degree of compatibility with the enzymatic step of the selection process. This approach further allows introducing larger chemical entities into DNA libraries that cannot be used using the methods of the state of the art. The method has a broad acceptance of diverse azide substrates, which are either commercially available or accessible by chemical synthesis.

The method further can comprise displacing one strand of at least one nucleic acid amplified in step (d), and further modifying the nucleic acid to introduce another functionalization.

In the method of the present invention, the candidate mixture comprises at least one nucleobase that is modified to comprise an azide-alkyne chemical group.

In embodiments, the azide-alkyne modified nucleobase comprises aliphatic-, aromatic-, charged-, basic-, acidic, heteroaromatic-, sugar-kind of-, metal-containing- or peptide- residues.

In embodiments, the nucleobase that is to be modified to contain an azide-alkyne chemical group is an ethynyl-, propynyl- or butynyl- dU, dA, dC or dG nucleotide.

In the method of the present invention, the amplification step (d) employs a polymerase chain reaction using an alkyne-modified nucleobase that is further modified via a 1,3 dipolar cycloaddition of an azide to yield an azide-alkyne modified nucleobase.

In embodiments, the azide comprises a light-sensitive molecule.

In embodiments, the target sample is selected from the group consisting of a polypeptide, peptide, cell, cell fragment, cell membrane, small organic molecule, cell culture, tissue sample, microvesicle, biological fluid sample and combination thereof.

In embodiments, the candidate mixture of nucleic acids in (a) is at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, at least 99%, or 100% modified to comprise the functionalization introduced by click chemistry.

In accordance with another aspect of the present disclosure is provided a method of detecting a presence or level of a target in a sample, comprising contacting at least one aptamer with the sample and detecting a binding event between the target in the sample and the at least one aptamer, wherein the at least one aptamer is identified or produced by the method of any claim.

In embodiments, the detecting comprises performing an immunoassay, ELISA, Western blot, sandwich assay, immunoprecipitation, affinity chromotography, flow cytometry, microbead assay, proteomic array, sequencing, or a combination thereof.

In embodiments, the presence of level of the target in the sample is compared to a reference level.

In embodiments, the comparison is used to characterize a phenotype in the sample.

In embodiments, the characterization comprises a diagnosis, prognosis or theranosis and the phenotype comprises the presence of a disease or disorder.

In embodiments, the disease or disorder comprises a cancer.

In accordance with another aspect of the present disclosure is provided a reagent comprising a nucleic acid ligand capable of binding a target sample, wherein the nucleic acid ligand comprises at least one nucleobase modified to contain an azide-alkyne chemical group.

In embodiments, the azide-alkyne chemical group comprises a light-sensitive molecule.

In embodiments, the azide-alkyne modified nucleobase comprises aliphatic-, aromatic-, charged-, basic-, acidic, heteroaromatic-, sugar-kind of-, metal-containing- or peptide- residues.

In embodiments, the nucleobase that is to be modified to contain an azide-alkyne chemical group is an ethynyl-, propynyl- or butynyl- dU, dA, dC or dG nucleotide.

In embodiments, the reagent is labeled with a gamma-emitting radioisotope.

In embodiments, an active agent is conjugated to the reagent.

In embodiments, the active agent comprises a therapeutic agent or a diagnostic agent.

In embodiments, the therapeutic agent or diagnostic agent is a therapeutic agent selected from the group consisting of tyrosine kinase inhibitors, kinase inhibitors, biologically active agents, biological molecules, radionuclides, adriamycin, ansamycin antibiotics, asparaginase, bleomycin, busulphan, cisplatin, carboplatin, carmustine, capecotabine, chlorambucil, cytarabine, cyclophosphamide, camptothecin, dacarbazine, dactinomycin, daunorubicin, dexrazoxane, docetaxel, doxorubicin, etoposide, epothilones, floxuridine, fludarabine, fluorouracil, gemcitabine, hydroxyurea, idarubicin, ifosfamide, irinotecan, lomustine, mechlorethamine, mercaptopurine, melphalan, methotrexate, rapamycin (sirolimus), mitomycin, mitotane, mitoxantrone, nitrosurea, paclitaxel, pamidronate, pentostatin, plicamycin, procarbazine, rituximab, streptozocin, teniposide, thioguanine, thiotepa, taxanes, vinblastine, vincristine, vinorelbine, taxol, combretastatins, discodermolides, transplatinum, anti-vascular endothelial growth factor compounds ("anti-VEGFs"), anti-epidermal growth factor receptor compounds ("anti-EGFRs"), 5-fluorouracil and derivatives, radionuclides, polypeptide toxins, apoptosis inducers, therapy sensitizers, enzyme or active fragment thereof, and combinations thereof.

In accordance with another aspect of the present disclosure is provided the use of the reagent to carry out the method of detecting a presence or level of a target in a sample.

In accordance with another aspect of the present disclosure is provided a pharmaceutical composition comprising a therapeutically effective amount of the reagent or a salt thereof, and a pharmaceutically acceptable carrier or diluent.

In accordance with another aspect of the present disclosure is provided the pharmaceutical composition comprising a therapeutically effective amount of the reagent or a salt thereof, and a pharmaceutically acceptable carrier or diluent for use in the treatment of or for ameliorating a disease or disorder or is provided a method of treating or ameliorating a disease or disorder, comprising administering the pharmaceutical composition to a subject in need thereof.

In embodiments, administering a therapeutically effective amount of the composition to the subject results in: (a) an enhancement of the delivery of the active agent to a disease site relative to delivery of the active agent alone; or (b) an enhancement of target clearance resulting in a decrease of at least 10%, 20%, 30%, 40%, 50%, 60%, 70%, 80%, or 90% in a blood level of the target; or (c) an decrease in biological activity of the target of the reagent by at least 10%, 20%, 30%, 40%, 50%, 60%, 70%, 80%, or 90%.

In embodiments, the target comprises a microvesicle population and the biological activity of the microvesicle population comprises immune suppression or transfer of genetic information.

In embodiments, the disease or disorder comprises a neoplastic, proliferative, inflammatory, metabolic, cardiovascular, or neurological disease or disorder.

Other features and advantages of the present invention will be apparent from the detailed description of the invention and from the claims.

### BRIEF DESCRIPTION OF THE DRAWINGS

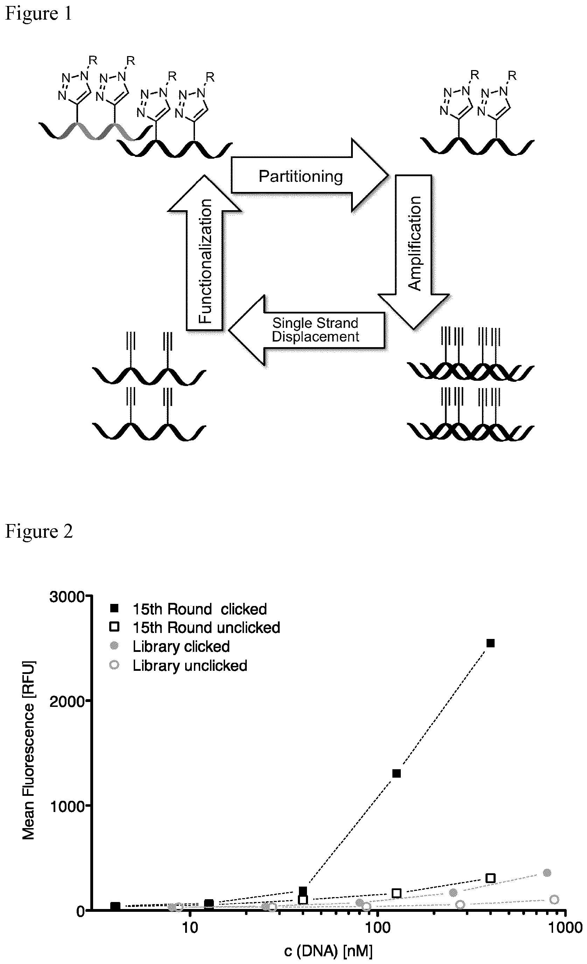

Figure 1 shows a schematic representation of an embodiment of the method.
Figure 2 shows the results of a FACS binding assay between the 15^{th} round enriched library and GFP-coated magnetic beads. The Cy5-labelled, 15^{th} round library leads to a concentration dependent, much higher mean fluorescence intensity on GFP-beads compared to the starting library. This increase in click dependent binding affinity demonstrates the successful enrichment of GFP binding sequences after 15 rounds of click SELEX.

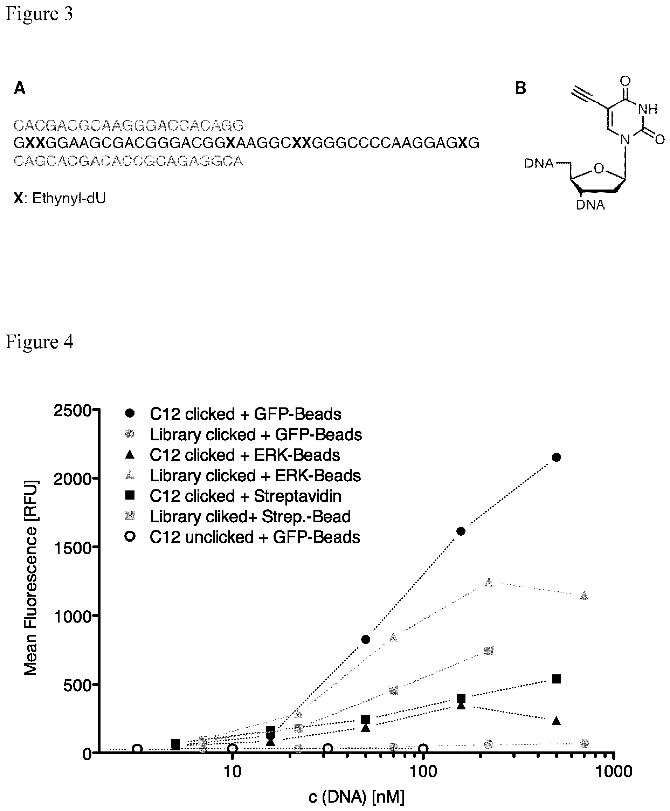

Figure 3-A) shows "C12" a monoclonal sequence isolated from the 15^{th} round enriched library, containing six ethynyl-dU (EdU) clickable nucleotides. Figure 7-B shows schematically the chemical structure of one ethynyl-dU nucleotide incorporated in a DNA strand.
Figure 4 shows the results of a FACS binding assay between the "C12" monoclonal sequence depicted in figure 7-A and protein-coated magnetic beads. C12 leads to a concentration dependent, much higher mean fluorescence intensity on the GFP-beads compared to the starting library. However, on ERK2 or streptavidin coated magnetic beads the situation is reversed and C12 shows less mean fluorescence intensity than the start library. This shows C12s high specificity to GFP compared to the two control proteins.

### DEFINITIONS

As used herein, the term "aptamer" refers to a small single-stranded oligonucleotide that recognises its target with high specificity and binds to the target with high affinity.

As used herein, the term "alkyne" refers to an unsaturated carbon moiety that has a carbon carbon triple bond between two carbon atoms.

As used herein, the term "azide" refers to a functional group RN₃. The group R refers to a chemical modification or additional chemical entity that can be introduced into the DNA. For example the group R can be a bulky photo cleavable chemical entity.

As used herein, the term "azide-alkyne chemical group" refers to a reaction product of an alkyne and an azide. The reaction can be an 1,3-dipolar cycloaddition of the azide with the alkyne. Via 1,3-dipolar cycloaddition of an azide with an alkyne the azide-alkyne linkeage can be a triazole linkage.

As used herein, the term "click chemistry" refers to bio-orthogonal reactions known to those of ordinary skill in the art. The term "click chemistry" can refer to a 1,3-dipolar cycloaddition. An example of click chemistry is the 1,3-dipolar cycloaddition of an azide to an alkyne, for example using the Cu(I) catalyzed 1,3-dipolar cycloaddition of an azides with an alkyne (CuAAC), or the copper-free strain-promoted azide-alkyne cycloaddition (SPAAC).

As used herein, the term "candidate mixture" refers to a mixture of nucleic acids of differing sequence, from which to select a desired ligand. The source of a candidate mixture can be from naturally-occurring nucleic acids or fragments thereof, chemically synthesized nucleic acids, enzymatically synthesized nucleic acids or nucleic acids made by a combination of the foregoing techniques.

As used herein, the term "ligand" refers to a nucleic acid that binds another molecule, e.g. the target. In a mixture of candidate nucleic acids, a ligand binds with greater affinity than that of the bulk mixture. In a candidate mixture can be comprised more than one ligand for a given target. The ligands can differ from one another in their binding affinities for the target.

As used herein, the term "nucleobase" refers to the nitrogen-containing biological compounds found within the DNA and RNA nucleotides and nucleotides, usually referred to as bases. The primary nucleobases are cytosine (C), guanine (G), adenine (A), thymine (T), and uracil (U).

As used herein, the term "nucleotide" refers to the basic building blocks of nucleic acids comprising or consisting of a nucleobase bound, ribose or deoxyribose and at least one phosphate group.

If not stated otherwise, the term "nucleic acid" refers to a ribonucleic acid or a deoxyribonucleic acid, single-stranded or double stranded. The aptamer according to the invention hence can be provided in the form of a single-stranded DNA or RNA molecule.

As used herein, the term "target sample" refers to any compound of interest for which an aptamer is desired. A sample can be any material, which probably contains a target to be determined, including any liquid or fluid sample or solid material, including a sample derived from a biological source. The term sample refers to polypeptides, peptides, or small organic molecules. The term sample also refers to biological material, for example cells, cell organelles, or tissues, or extracts of any of the foregoing, biological fluids, biological molecules, or supernatants.

As used herein, the term "amplifying" refers to any process or combination of process steps that increases the amount or number of copies of a molecule such as a nucleic acid. Polymerase chain reaction (PCR) is an exemplary method for amplifying of nucleic acids.

As used herein, the term "partitioning" refers to any process whereby aptamers bound to the target sample can be separated from nucleic acids not bound to the target. Partitioning for example can be accomplished by immobilization of the nucleic acids bound to the target on magnetic beads or by capillary electrophoresis of the bound nucleic acids against the target. The choice of partitioning method will depend on properties of the target sample and of the nucleic acids that bind to the target sample and can be made according to principles and properties known to those of ordinary skill in the art.

Unless defined otherwise, the technical and scientific terms used herein have the same meaning as commonly understood by one of ordinary skill in the art to which the invention belongs. Particularly, unless otherwise stated, a term as used herein is given the definition as provided in the Oxford dictionary of biochemistry and molecular biology, Oxford University Press, 1997, revised 2000 and reprinted 2003, ISBN 0 19 850673 2.

It should be noted that, as used in this specification and the appended claims, the singular forms "a", "an" and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

### DETAILED DESCRIPTION OF THE INVENTION

According to the invention it is provided a method of identifying or producing an aptamer comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the nucleobase that has been modified to contain the azide-alkyne group was an ethynyl-dU, dA, dC or dG nucleotide;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer,wherein the binding nucleic acids are amplified by PCR using an ethynyl-modified nucleobase and again are subjected to an in vitro selection cycle until an enrichment of target-binding nucleic acids is detected.

The method enables the introduction of additional chemical entities into DNA during the selection process. Advantageously, the modification is only transiently present during the selection cycle. This enables a high degree of compatibility with the enzymatic step of the selection process. This approach further allows introducing larger chemical entities into DNA libraries that cannot be used using known methods. The method has a broad acceptance of diverse azide substrates, which are either commercially available or accessible by chemical synthesis.

In an embodiment, the method further comprises displacing one strand of at least one nucleic acid amplified in step (d), and further modifying the nucleic acid to introduce another functionalization.

A DNA library modified to comprise a functionalization, e.g. an azide-alkyne chemical group, can be incubated with a target molecule. After incubation, the unbound sequences will be removed and the bound molecules recovered and amplified by PCR. The method of displacing one strand of the nucleic acid can be made according to principles and properties known to those of ordinary skill in the art. Single-stranded DNA can be achieved by selective lambda exonuclease digestion of one strand. The resultant DNA then again can be modified by re-introducing the chemical modification. This library again can be subjected to an in vitro selection cycle until an enrichment of target-binding sequences can be detected.

In a first SELEX cycle, in step (a) a candidate mixture of nucleic acids can be provided that is prepared by chemical synthesis. When displacing one strand of at least one nucleic acid amplified in step (d), and further modifying the nucleic acid to introduce another functionalization, e.g. an azide-alkyne chemical group, in the following cycles the candidate mixtures can be prepared in a semi enzymatic way.

Particularly click chemistry on the basis of 1,3 dipolar cycloadditions is usable in aqueous biological systems to introduce chemical entities. Several types of reaction have been identified that are usable for click chemistry via 1,3 dipolar cycloaddition. Particularly useful has proven the 1,3 dipolar cycloaddition of an azide to an alkyne. Further, the modification of the nucleobase has proven useful in the method. Hence, the candidate mixture comprises at least one nucleobase that is modified to comprise an azide-alkyne chemical group.

In an embodiment, the amplification step (d) employs a polymerase chain reaction using an alkyne-modified nucleobase that is further modified via 1,3 dipolar cycloaddition of an azide to yield an azide-alkyne modified nucleobase.

The nucleic acids of the candidate mixture can be single-stranded DNA. The introduction of additional chemical entities into DNA during the selection process can be achieved by using a 5-alkyne modified nucleobase, for example thymine. 5-C8-alkyne modified nucleotide-triphosphates for example deoxythymidines are commercially available or can be synthesized. Such 5-C8-alkyne modified nucleobases for example deoxythymidines can be introduced into DNA by PCR. The modification can be further derivatized with so called bio-orthogonal chemistry, for example using the Cu(I) catalyzed 1,3-dipolar cycloaddition of respective azides with the alkyne. Beside the Cu(I) catalysed azide-alkyne cycloaddition (CuAAC), copper-free strain-promoted azide-alkyne cycloaddition (SPAAC) reactions also are usable. In applications in cellular or living systems the strain-promoted azide-alkyne cycloaddition can overcome toxicity issues associated with the use of Cu(I).

Any number of desirable chemical modifications can be added to the oligonucleotide library used for screening purposes. Examples of such modifications include without limitation aliphatic-aromatic-, charged-, basic-, acidic, heteroaromatic-, sugar-kind of-, metal-containing- or peptide-residues.

The nucleobase that is to be modified to contain an azide-alkyne chemical group is an ethynyl-dU, dA, dC or dG nucleotide. The nucleobase that is to be modified to contain an azide-alkyne chemical group may be an ethynyl-dU nucleotide, or an ethynyl-dA nucleotide, an ethynyl-dC nucleotide or an ethynyl-dG nucleotide.

DNA libraries that are modified in this way can then be employed for in vitro selection purposes. Hence, this reaction enables to introduce chemical modifications into DNA applicable in SELEX. Thereby, the chemical diversity of DNA libraries can be enhanced.

The starting, or candidate, mixture of nucleic acids can be modified such that at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, at least 99%, or 100% of the members of the mixture are modified to comprise the functionalization introduced by click chemistry. Less than 100% modification may allow for enhanced diversity by allowing certain positions in an oligonucleotide to be modified but not others, whereas 100% modification ensures consistency during the selection process. In some embodiments, different modifications are made at different positions in the oligonucleotide to further enhance diversity.

In an embodiment, the azide can be modified with a light-sensitive molecule. The use of photosensitive modifications further enhances the approach towards bulky residues to be introduced and used in the selection process. It also enables the direct selection of photoresponsive aptamers. This allows the development of entirely new selection protocols.

The light-sensitive molecule can be a bulky group that inhibits amplification by PCR. When comprising a photo cleavable moiety, a cleavage of the bulky group can be initiated by irradiation, for example with ultraviolet light, and the remaining smaller azide-alkyne modified molecule can become available to PCR.

The method can be applied to a broad variety of target samples. This allows for a wide diversity of samples including biological structures. In an embodiment, the target sample is selected from the group consisting of a polypeptide, peptide, protein, cell, cell fragment, cell membrane, microvesicle, small organic molecule, biological fluid sample and combination thereof. Especially, the method is not limited to purified samples, of polypeptides, peptides or small organic molecules, but extendable to highly complex biological contexts such as biological fluid sample or even directly at the membranes of cells.

Another aspect, the disclosure relates to a reagent comprising a nucleic acid ligand capable of binding a target sample, wherein the nucleic acid ligand comprises at least one nucleobase modified to contain an azide-alkyne chemical group.

The modified nucleobase for example can the thymine. The modification can be achieved by using a 5-C8-alkyne modified deoxythymidine that can be introduced into DNA by PCR. The modification can be further derivatized with socalled bio-orthogonal chemistry, for example using the Cu(I) catalyzed 1,3-dipolar cycloaddition of a respective azides with the alkyne.

In an embodiment, the azide-alkyne chemical group comprises a light-sensitive molecule. The light-sensitive molecule can be a bulky group that inhibits PCR amplification of the nucleic acid ligand. Upon initiation of cleavage of a photo cleavable moiety by irradiation with ultraviolet light, a remaining smaller azide-alkyne modified molecule allows amplification.

The invention will be explained in more detail by virtue of the examples set forth herein below. While at least one exemplary embodiment is presented, it should be appreciated that a vast number of variations exist. It should also be appreciated that the exemplary embodiments are only examples, and are not intended to limit the scope, applicability, or configuration in any way. Rather, the forgoing description will provide those with ordinary skill in the art with the essential characteristics of this invention for implementing at least one exemplary embodiment, it being understood that various changes may be made without departing from the scope as set forth in the appended claims.

The figure 1 shows a schematic representation of an embodiment of the method. A DNA library modified to comprise an azide-alkyne group is incubated with a target molecule. After incubation, the unbound sequences are removed and the bound molecules recovered. The selected molecule is amplified by PCR, using an alkyne-modified nucleobase. Single-strand displacement is achieved by selective lambda exonuclease digestion of one strand. The resultant DNA is then again modified by 1,3-dipolar cycloaddition of an azide RN₃, thereby re-introducing the chemical modification of an azide-alkyne group. This library is again subjected to an in vitro selection cycle until an enrichment of target-binding sequences can be detected.

### Biomarker Detection

The click-modified aptamers of the present disclosure can be used in various methods to detect a presence or level of one or more biomarker in a biological sample, e.g., biological entities of interest such as proteins, nucleic acids, or microvesicles. The aptamer can function as a binding agent to assess presence or level of the cognate target molecule. In various embodiments of the disclosure directed to diagnostics, prognostics or theranostics, one or more aptamers are configured in a ligand-target based assay, where one or more aptamer is contacted with a selected biological sample to allow the or more aptamer to associate with or binds to its target molecules. Aptamers are used to identify a profile of multiple biomarkers (a "biomarker" profile) based on the biological samples assessed and biomarkers detected. A biomarker profile of a biological sample may comprise a presence, level or other characteristic of one or more biomarker of interest that can be assessed, including without limitation a presense, level, sequence, mutation, rearrangement, translocation, deletion, epigenetic modification, methylation, post-translational modification, allele, activity, complex partners, stability, half life, and the like.

Biomarker profiles can be used to evaluate diagnostic and/or prognostic criteria such as presence of disease, disease staging, disease monitoring, disease stratification, or surveillance for detection, metastasis or recurrence or progression of disease. For example, methods using the click-modified aptamers against microvesicle surface antigens are useful for correlating a biomarker profile comprising microvesicle antigens to a selected condition or disease. A biomarker profile can also be used clinically in making decisions concerning treatment modalities including therapeutic intervention. A biomarker profile can further be used clinically to make treatment decisions, including whether to perform surgery or what treatment standards should be used along with surgery (e.g., either pre-surgery or post-surgery). As an illustrative example, a biomarker profile of circulating biomarkers that indicates an aggressive form of cancer may call for a more aggressive surgical procedure and/or more aggressive therapeutic regimen to treat the patient.

A biomarker profile such as determined in whole or in part using an aptamer provided herein can be used in any methods disclosed herein, e.g., to assess whether a subject is afflicted with disease, is at risk for developing disease or to assess the stage or progression of the disease. For example, a biomarker profile can be used to assess whether a subject has prostate cancer, colon cancer, or other cancer as described herein. Furthermore, a biomarker profile can be used to determine a stage of a disease or condition.

A biomarker profile comprising a microvesicle can include assessment of payload within the microvesicle. For example, one or more click-modified aptamer can be used to capture a microvesicle population using a specific microvesicle associated surface antigen, and then the payload content within the captured microvesicles can be assessed, thereby providing further biomarker readout of the payload content.

### Phenotypes

As noted above, one or more click-modified aptamer can be used for characterizing a phenotype of interest in a sample. The term "phenotype" as used herein can mean any trait or characteristic that is attributed to a biomarker profile that is identified using in part or in whole the compositions and/or methods of the present disclosure. For example, a phenotype can be a diagnostic, prognostic or theranostic determination based on a characterized biomarker profile for a sample obtained from a subject. A phenotype can be any observable characteristic or trait of, such as a disease or condition, a stage of a disease or condition, susceptibility to a disease or condition, prognosis of a disease stage or condition, a physiological state, or response / potential response to therapeutics. A phenotype can result from a subject's genetic makeup as well as the influence of environmental factors and the interactions between the two, as well as from epigenetic modifications to nucleic acid sequences.

A phenotype in a subject can be characterized by obtaining a biological sample from a subject and analyzing the sample using one or more click-modified aptamer. For example, characterizing a phenotype for a subject or individual can include detecting a disease or condition (including pre-symptomatic early stage detecting), determining a prognosis, diagnosis, or theranosis of a disease or condition, or determining the stage or progression of a disease or condition. Characterizing a phenotype can include identifying appropriate treatments or treatment efficacy for specific diseases, conditions, disease stages and condition stages, predictions and likelihood analysis of disease progression, particularly disease recurrence, metastatic spread or disease relapse. A phenotype can also be a clinically distinct type or subtype of a condition or disease, such as a cancer or tumor. Phenotype determination can also be a determination of a physiological condition, or an assessment of organ distress or organ rejection, such as post-transplantation. The compositions and methods described herein allow assessment of a subject on an individual basis, which can provide benefits of more efficient and economical decisions in treatment. For example, the click-modified aptamers can be used in an assay to characterize a phenotype.

Theranostics includes diagnostic testing that provides the ability to affect therapy or treatment of a disease or disease state. Theranostics testing provides a theranosis in a similar manner that diagnostics or prognostic testing provides a diagnosis or prognosis, respectively. As used herein, theranostics encompasses any desired form of therapy related testing, including predictive medicine, personalized medicine, integrated medicine, pharmacodiagnostics and Dx/Rx partnering. Therapy related tests can be used to predict and assess drug response in individual subjects, i.e., to provide personalized medicine. Predicting a drug response can be determining whether a subject is a likely responder or a likely non-responder to a candidate therapeutic agent, e.g., before the subject has been exposed or otherwise treated with the treatment. Assessing a drug response can be monitoring a response to a drug, e.g., monitoring the subject's improvement or lack thereof over a time course after initiating the treatment. Therapy related tests are useful to select a subject for treatment who is particularly likely to benefit from the treatment or to provide an early and objective indication of treatment efficacy in an individual subject. Thus, analysis using the compositions and methods of the present disclosure may indicate that treatment should be altered to select a more promising treatment, thereby avoiding the great expense of delaying beneficial treatment and avoiding the financial and morbidity costs of administering an ineffective drug(s).

The click-modified aptamers may be used to provide a theranostic readout, including without limitation predicting the responder / non-responder status of the subject, wherein a responder responds to a treatment for a disease and a non-responder does not respond to the treatment. Biomarkers such as proteins and microvesicles can be analyzed in the subject and compared against that of previous subjects that were known to respond or not to a treatment. If the biomarker profile in the subject more closely aligns with that of previous subjects that were known to respond to the treatment, the subject can be characterized, or predicted, as a responder to the treatment. Similarly, if the biomarker profile in the subject more closely aligns with that of previous subjects that did not respond to the treatment, the subject can be characterized, or predicted as a non-responder to the treatment. The treatment can be for any appropriate disease, disorder or other condition, including without limitation those disclosed herein.

A biomarker profile for characterizing a phenotype may comprise any number of useful criteria. A phenotype can be detected or identified in part or in whole using the compositions and/or methods of the present disclosure. In some embodiments, at least one criterion is used to characterize a phenotype of interest. In some embodiments, at least 1, 2, 3, 4, 5, 6, 7, 8, 9, 10, 15, 20, 30, 40, 50, 60, 70, 80, 90 or at least 100 criteria are used. For example, for the characterizing of a cancer, a number of different criteria can be used when the subject is diagnosed with a cancer: 1) if the amount of a biomarker in a sample from a subject is higher than a reference value; 2) if the amount of a biomarker within specific cells, tissue or derivatives thereof (e.g., vesicles derived from a specific tissue or organ) is higher than a reference value; or 3) if the amount of a biomarker within vesicles with one or more cancer specific biomarkers is higher than a reference value. Similar rules can apply if the amount of a biomarker is less than or the same as the reference. The method can further include a quality control measure, such that the results are provided for the subject if the samples meet the quality control measure. In some embodiments, if the criteria are met but the quality control is questionable, the subject is reassessed.

The click-modified aptamers can be used to assist in characterizing various phenotypes of interest. For example, the phenotype can comprise detecting the presence of or likelihood of developing a tumor, neoplasm, or cancer, or characterizing the tumor, neoplasm, or cancer (e.g., stage, grade, aggressiveness, likelihood of metastatis or recurrence, etc). Examplary phenotypes are presented below.

The phenotype can be a premalignant condition, such as actinic keratosis, atrophic gastritis, leukoplakia, erythroplasia, Lymphomatoid Granulomatosis, preleukemia, fibrosis, cervical dysplasia, uterine cervical dysplasia, xeroderma pigmentosum, Barrett's Esophagus, colorectal polyp, or other abnormal tissue growth or lesion that is likely to develop into a malignant tumor. Transformative viral infections such as HIV and HPV also present phenotypes that can be assessed.

A cancer characterized by the methods of the present disclosure can comprise, without limitation, a carcinoma, a sarcoma, a lymphoma or leukemia, a germ cell tumor, a blastoma, or other cancers. Carcinomas include without limitation epithelial neoplasms, squamous cell neoplasms squamous cell carcinoma, basal cell neoplasms basal cell carcinoma, transitional cell papillomas and carcinomas, adenomas and adenocarcinomas (glands), adenoma, adenocarcinoma, linitis plastica insulinoma, glucagonoma, gastrinoma, vipoma, cholangiocarcinoma, hepatocellular carcinoma, adenoid cystic carcinoma, carcinoid tumor of appendix, prolactinoma, oncocytoma, hurthle cell adenoma, renal cell carcinoma, grawitz tumor, multiple endocrine adenomas, endometrioid adenoma, adnexal and skin appendage neoplasms, mucoepidermoid neoplasms, cystic, mucinous and serous neoplasms, cystadenoma, pseudomyxoma peritonei, ductal, lobular and medullary neoplasms, acinar cell neoplasms, complex epithelial neoplasms, warthin's tumor, thymoma, specialized gonadal neoplasms, sex cord stromal tumor, thecoma, granulosa cell tumor, arrhenoblastoma, sertoli leydig cell tumor, glomus tumors, paraganglioma, pheochromocytoma, glomus tumor, nevi and melanomas, melanocytic nevus, malignant melanoma, melanoma, nodular melanoma, dysplastic nevus, lentigo maligna melanoma, superficial spreading melanoma, and malignant acral lentiginous melanoma. Sarcoma includes without limitation Askin's tumor, botryodies, chondrosarcoma, Ewing's sarcoma, malignant hemangio endothelioma, malignant schwannoma, osteosarcoma, soft tissue sarcomas including: alveolar soft part sarcoma, angiosarcoma, cystosarcoma phyllodes, dermatofibrosarcoma, desmoid tumor, desmoplastic small round cell tumor, epithelioid sarcoma, extraskeletal chondrosarcoma, extraskeletal osteosarcoma, fibrosarcoma, hemangiopericytoma, hemangiosarcoma, kaposi's sarcoma, leiomyosarcoma, liposarcoma, lymphangiosarcoma, lymphosarcoma, malignant fibrous histiocytoma, neurofibrosarcoma, rhabdomyosarcoma, and synovialsarcoma. Lymphoma and leukemia include without limitation chronic lymphocytic leukemia/small lymphocytic lymphoma, B-cell prolymphocytic leukemia, lymphoplasmacytic lymphoma (such as waldenstrom macroglobulinemia), splenic marginal zone lymphoma, plasma cell myeloma, plasmacytoma, monoclonal immunoglobulin deposition diseases, heavy chain diseases, extranodal marginal zone B cell lymphoma, also called malt lymphoma, nodal marginal zone B cell lymphoma (nmzl), follicular lymphoma, mantle cell lymphoma, diffuse large B cell lymphoma, mediastinal (thymic) large B cell lymphoma, intravascular large B cell lymphoma, primary effusion lymphoma, burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides / sezary syndrome, primary cutaneous CD30-positive T cell lymphoproliferative disorders, primary cutaneous anaplastic large cell lymphoma, lymphomatoid papulosis, angioimmunoblastic T cell lymphoma, peripheral T cell lymphoma, unspecified, anaplastic large cell lymphoma, classical hodgkin lymphomas (nodular sclerosis, mixed cellularity, lymphocyte-rich, lymphocyte depleted or not depleted), and nodular lymphocyte-predominant hodgkin lymphoma. Germ cell tumors include without limitation germinoma, dysgerminoma, seminoma, nongerminomatous germ cell tumor, embryonal carcinoma, endodermal sinus turmor, choriocarcinoma, teratoma, polyembryoma, and gonadoblastoma. Blastoma includes without limitation nephroblastoma, medulloblastoma, and retinoblastoma. Other cancers include without limitation labial carcinoma, larynx carcinoma, hypopharynx carcinoma, tongue carcinoma, salivary gland carcinoma, gastric carcinoma, adenocarcinoma, thyroid cancer (medullary and papillary thyroid carcinoma), renal carcinoma, kidney parenchyma carcinoma, cervix carcinoma, uterine corpus carcinoma, endometrium carcinoma, chorion carcinoma, testis carcinoma, urinary carcinoma, melanoma, brain tumors such as glioblastoma, astrocytoma, meningioma, medulloblastoma and peripheral neuroectodermal tumors, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, and plasmocytoma.

In embodiments, the cancer comprises an acute lymphoblastic leukemia; acute myeloid leukemia; adrenocortical carcinoma; AIDS-related cancers; AIDS-related lymphoma; anal cancer; appendix cancer; astrocytomas; atypical teratoid/rhabdoid tumor; basal cell carcinoma; bladder cancer; brain stem glioma; brain tumor (including brain stem glioma, central nervous system atypical teratoid/rhabdoid tumor, central nervous system embryonal tumors, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma); breast cancer; bronchial tumors; Burkitt lymphoma; cancer of unknown primary site; carcinoid tumor; carcinoma of unknown primary site; central nervous system atypical teratoid/rhabdoid tumor; central nervous system embryonal tumors; cervical cancer; childhood cancers; chordoma; chronic lymphocytic leukemia; chronic myelogenous leukemia; chronic myeloproliferative disorders; colon cancer; colorectal cancer; craniopharyngioma; cutaneous T-cell lymphoma; endocrine pancreas islet cell tumors; endometrial cancer; ependymoblastoma; ependymoma; esophageal cancer; esthesioneuroblastoma; Ewing sarcoma; extracranial germ cell tumor; extragonadal germ cell tumor; extrahepatic bile duct cancer; gallbladder cancer; gastric (stomach) cancer; gastrointestinal carcinoid tumor; gastrointestinal stromal cell tumor; gastrointestinal stromal tumor (GIST); gestational trophoblastic tumor; glioma; hairy cell leukemia; head and neck cancer; heart cancer; Hodgkin lymphoma; hypopharyngeal cancer; intraocular melanoma; islet cell tumors; Kaposi sarcoma; kidney cancer; Langerhans cell histiocytosis; laryngeal cancer; lip cancer; liver cancer; malignant fibrous histiocytoma bone cancer; medulloblastoma; medulloepithelioma; melanoma; Merkel cell carcinoma; Merkel cell skin carcinoma; mesothelioma; metastatic squamous neck cancer with occult primary; mouth cancer; multiple endocrine neoplasia syndromes; multiple myeloma; multiple myeloma/plasma cell neoplasm; mycosis fungoides; myelodysplastic syndromes; myeloproliferative neoplasms; nasal cavity cancer; nasopharyngeal cancer; neuroblastoma; Non-Hodgkin lymphoma; nonmelanoma skin cancer; non-small cell lung cancer; oral cancer; oral cavity cancer; oropharyngeal cancer; osteosarcoma; other brain and spinal cord tumors; ovarian cancer; ovarian epithelial cancer; ovarian germ cell tumor; ovarian low malignant potential tumor; pancreatic cancer; papillomatosis; paranasal sinus cancer; parathyroid cancer; pelvic cancer; penile cancer; pharyngeal cancer; pineal parenchymal tumors of intermediate differentiation; pineoblastoma; pituitary tumor; plasma cell neoplasm/multiple myeloma; pleuropulmonary blastoma; primary central nervous system (CNS) lymphoma; primary hepatocellular liver cancer; prostate cancer; rectal cancer; renal cancer; renal cell (kidney) cancer; renal cell cancer; respiratory tract cancer; retinoblastoma; rhabdomyosarcoma; salivary gland cancer; Sézary syndrome; small cell lung cancer; small intestine cancer; soft tissue sarcoma; squamous cell carcinoma; squamous neck cancer; stomach (gastric) cancer; supratentorial primitive neuroectodermal tumors; T-cell lymphoma; testicular cancer; throat cancer; thymic carcinoma; thymoma; thyroid cancer; transitional cell cancer; transitional cell cancer of the renal pelvis and ureter; trophoblastic tumor; ureter cancer; urethral cancer; uterine cancer; uterine sarcoma; vaginal cancer; vulvar cancer; Waldenstrom macroglobulinemia; or Wilm's tumor.

In some embodiments, the cancer comprises an acute myeloid leukemia (AML), breast carcinoma, cholangiocarcinoma, colorectal adenocarcinoma, extrahepatic bile duct adenocarcinoma, female genital tract malignancy, gastric adenocarcinoma, gastroesophageal adenocarcinoma, gastrointestinal stromal tumors (GIST), glioblastoma, head and neck squamous carcinoma, leukemia, liver hepatocellular carcinoma, low grade glioma, lung bronchioloalveolar carcinoma (BAC), lung non-small cell lung cancer (NSCLC), lung small cell cancer (SCLC), lymphoma, male genital tract malignancy, malignant solitary fibrous tumor of the pleura (MSFT), melanoma, multiple myeloma, neuroendocrine tumor, nodal diffuse large B-cell lymphoma, non epithelial ovarian cancer (non-EOC), ovarian surface epithelial carcinoma, pancreatic adenocarcinoma, pituitary carcinomas, oligodendroglioma, prostatic adenocarcinoma, retroperitoneal or peritoneal carcinoma, retroperitoneal or peritoneal sarcoma, small intestinal malignancy, soft tissue tumor, thymic carcinoma, thyroid carcinoma, or uveal melanoma. The click-modified aptamer of the present disclosure can be used to characterize these and other cancers.

Thus, characterizing a phenotype can be providing a diagnosis, prognosis or theranosis of one of the cancers disclosed herein.

The phenotype can also be an inflammatory disease, immune disease, or autoimmune disease. For example, the disease may be inflammatory bowel disease (IBD), Crohn's disease (CD), ulcerative colitis (UC), pelvic inflammation, vasculitis, psoriasis, diabetes, autoimmune hepatitis, Multiple Sclerosis, Myasthenia Gravis, Type I diabetes, Rheumatoid Arthritis, Psoriasis, Systemic Lupus Erythematosis (SLE), Hashimoto's Thyroiditis, Grave's disease, Ankylosing Spondylitis Sjogrens Disease, CREST syndrome, Scleroderma, Rheumatic Disease, organ rejection, Primary Sclerosing Cholangitis, or sepsis.

The phenotype can also comprise a cardiovascular disease, such as atherosclerosis, congestive heart failure, vulnerable plaque, stroke, or ischemia. The cardiovascular disease or condition can be high blood pressure, stenosis, vessel occlusion or a thrombotic event.

The phenotype can also comprise a neurological disease, such as Multiple Sclerosis (MS), Parkinson's Disease (PD), Alzheimer's Disease (AD), schizophrenia, bipolar disorder, depression, autism, Prion Disease, Pick's disease, dementia, Huntington disease (HD), Down's syndrome, cerebrovascular disease, Rasmussen's encephalitis, viral meningitis, neurospsychiatric systemic lupus erythematosus (NPSLE), amyotrophic lateral sclerosis, Creutzfeldt-Jacob disease, Gerstmann-Straussler-Scheinker disease, transmissible spongiform encephalopathy, ischemic reperfusion damage (e.g. stroke), brain trauma, microbial infection, or chronic fatigue syndrome.

The phenotype may also be a condition such as fibromyalgia, chronic neuropathic pain, or peripheral neuropathic pain.

The phenotype may also comprise an infectious disease, such as a bacterial, viral or yeast infection. For example, the disease or condition may be Whipple's Disease, Prion Disease, cirrhosis, methicillin-resistant staphylococcus aureus, HIV, hepatitis, syphilis, meningitis, malaria, tuberculosis, or influenza. Viral proteins, such as HIV or HCV-like particles can be assessed in a vesicle, to characterize a viral condition.

The phenotype can also comprise a perinatal or pregnancy related condition (e.g. preeclampsia or preterm birth), metabolic disease or condition, such as a metabolic disease or condition associated with iron metabolism. For example, hepcidin can be assayed in a vesicle to characterize an iron deficiency. The metabolic disease or condition can also be diabetes, inflammation, or a perinatal condition.

The click-modified aptamers can be used to characterize these and other diseases and disorders that can be assessed via biomarkers, e.g., by assessing a biomarker using a click modified aptamer. Thus, characterizing a phenotype can be providing a diagnosis, prognosis or theranosis of one of the diseases and disorders disclosed herein.

### Subject

One or more phenotypes of a subject can be determined by analyzing a biomarker profile in a biological sample obtained from the subject. A subject, but is not limited to, mammals such as bovine, avian, canine, equine, feline, ovine, porcine, or primate animals (including humans and non-human primates). A subject can also include a mammal of importance due to being endangered, such as a Siberian tiger; or economic importance, such as an animal raised on a farm for consumption by humans, or an animal of social importance to humans, such as an animal kept as a pet or in a zoo. Examples of such animals include, but are not limited to, carnivores such as cats and dogs; swine including pigs, hogs and wild boars; ruminants or ungulates such as cattle, oxen, sheep, giraffes, deer, goats, bison, camels or horses. Also included are birds that are endangered or kept in zoos, as well as fowl and more particularly domesticated fowl, i.e. poultry, such as turkeys and chickens, ducks, geese, guinea fowl. Also included are domesticated swine and horses (including race horses). In addition, any animal species connected to commercial activities are also included such as those animals connected to agriculture and aquaculture and other activities in which disease monitoring, diagnosis, and therapy selection are routine practice in husbandry for economic productivity and/or safety of the food chain.

The subject can have a pre-existing disease or condition, such as cancer or other disease or disorder listed herein (see, e.g., section "Phenotypes"). Alternatively, the subject may not have any known pre-existing condition. The subject may also be non-responsive to an existing or past treatment, such as a treatment for cancer.

### Samples

A sample can include any relevant biological sample that can be used for biomarker assessment using a click modified aptamer, including without limitation sections of tissues such as biopsy or tissue removed during surgical or other procedures, bodily fluids, autopsy samples, frozen sections taken for histological purposes, and cell cultures. Such samples include blood and blood fractions or products (e.g., serum, buffy coat, plasma, platelets, red blood cells, and the like), sputum, malignant effusion, cheek cells tissue, cultured cells (e.g., primary cultures, explants, and transformed cells), stool, urine, other biological or bodily fluids (e.g., prostatic fluid, gastric fluid, intestinal fluid, renal fluid, lung fluid, cerebrospinal fluid, and the like), etc. The sample can comprise biological material that is a fresh frozen & formalin fixed paraffin embedded (FFPE) block, formalin-fixed paraffin embedded, or is within an RNA preservative + formalin fixative. More than one sample of more than one type can be used for each patient.

The sample used in the methods described herein can be a formalin fixed paraffin embedded (FFPE) sample. The FFPE sample can be one or more of fixed tissue, unstained slides, bone marrow core or clot, core needle biopsy, malignant fluids and fine needle aspirate (FNA). In an embodiment, the fixed tissue comprises a tumor containing formalin fixed paraffin embedded (FFPE) block from a surgery or biopsy. In another embodiment, the unstained slides comprise unstained, charged, unbaked slides from a paraffin block. In another embodiment, bone marrow core or clot comprises a decalcified core. A formalin fixed core and/or clot can be paraffin-embedded. In still another embodiment, the core needle biopsy comprises 1, 2, 3, 4, 5, 6, 7, 8, 9, 10 or more, e.g., 3-4, paraffin embedded biopsy samples. An 18 gauge needle biopsy can be used. The malignant fluid can comprise a sufficient volume of fresh pleural/ascitic fluid to produce a 5x5x2mm cell pellet. The fluid can be formalin fixed in a paraffin block. In an embodiment, the core needle biopsy comprises 1, 2, 3, 4, 5, 6, 7, 8, 9, 10 or more, e.g., 4-6, paraffin embedded aspirates.

A sample may be processed according to techniques understood by those in the art. A sample can be without limitation fresh, frozen or fixed cells or tissue. In some embodiments, a sample comprises formalin-fixed paraffin-embedded (FFPE) tissue, fresh tissue or fresh frozen (FF) tissue. A sample can comprise cultured cells, including primary or immortalized cell lines derived from a subject sample. A sample can also refer to an extract from a sample from a subject. For example, a sample can comprise DNA, RNA or protein extracted from a tissue or a bodily fluid. Many techniques and commercial kits are available for such purposes. The fresh sample from the individual can be treated with an agent to preserve RNA prior to further processing, e.g., cell lysis and extraction. Samples can include frozen samples collected for other purposes. Samples can be associated with relevant information such as age, gender, and clinical symptoms present in the subject; source of the sample; and methods of collection and storage of the sample. A sample may be obtained from a subject.

A biopsy comprises the process of removing a tissue sample for diagnostic or prognostic evaluation, and to the tissue specimen itself. Any biopsy technique known in the art can be applied to the biomarker profiling methods of the present disclosure. The biopsy technique applied can depend on the tissue type to be evaluated (e.g., colon, prostate, kidney, bladder, lymph node, liver, bone marrow, blood cell, lung, breast, etc.), the size and type of the tumor (e.g., solid or suspended, blood or ascites), among other factors. Representative biopsy techniques include, but are not limited to, excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue that includes a cross-sectional diameter of the tumor. Molecular profiling can use a "core-needle biopsy" of the tumor mass, or a "fine-needle aspiration biopsy" which generally obtains a suspension of cells from within the tumor mass.

Biopsy techniques are discussed, for example, in Harrison's Principles of Internal Medicine, Kasper, et al., eds., 16th ed., 2005, Chapter 70, and throughout Part V.

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998) and methodology as set forth in U.S. Pat. Nos. 4,666,828; 4,683,202; 4,801,531; 5,192,659 and 5,272,057. Polymerase chain reaction (PCR) can be carried out generally as in PCR Protocols: A Guide to Methods and Applications, Academic Press, San Diego, Calif. (1990).

The biological sample assessed using the compositions and methods can be any useful bodily or biological fluid, including but not limited to peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids, cells, cell culture, or a cell culture supernatant. A biological sample may also include the blastocyl cavity, umbilical cord blood, or maternal circulation which may be of fetal or maternal origin. The biological sample may also be a cell culture, tissue sample or biopsy from which vesicles and other circulating biomarkers may be obtained. For example, cells of interest can be cultured and vesicles or other biomarkers isolated from the culture. In various embodiments, biomarker profiles can be assessed directly from such biological samples (e.g., identification of presence or levels of nucleic acid or polypeptide biomarkers or functional fragments thereof) using various methods, such as extraction of nucleic acid molecules from blood, plasma, serum or any of the foregoing biological samples, use of protein or antibody arrays to identify polypeptide (or functional fragment) biomarker(s), as well as other array, sequencing, PCR and proteomic techniques known in the art for identification and assessment of nucleic acid and polypeptide molecules. In addition, one or more components present in such samples can be first isolated or enriched and further processed to assess the presence or levels of selected biomarkers, to assess a given biomarker profile (e.g., isolated microvesicles prior to profiling for protein and/or nucleic acid biomarkers).

**Table 1** presents a non-limiting listing of diseases, conditions, or biological states and corresponding biological samples that may be used for analysis according to the methods of the present disclosure.

**Table 1: Examples of Biological Samples**

| **Illustrative Disease, Condition or Biological State** | **Illustrative Biological Samples** |
|---|---|
| *Cancers*/*neoplasms affecting the following tissue types*/*bodily systems:* breast, lung, ovarian, colon, rectal, prostate, pancreatic, brain, bone, connective tissue, glands, skin, lymph, nervous system, endocrine, germ cell, genitourinary, hematologic/blood, bone marrow, muscle, eye, esophageal, fat tissue, thyroid, pituitary, spinal cord, bile duct, heart, gall bladder, bladder, testes, cervical, endometrial, renal, ovarian, digestive/gastrointestinal, stomach, head and neck, liver, leukemia, respiratory/thorasic, cancers of unknown primary (CUP) | Tumor, blood, serum, plasma, cerebrospinal fluid (CSF), urine, sputum, ascites, synovial fluid, semen, nipple aspirates, saliva, bronchoalveolar lavage fluid, tears, oropharyngeal washes, feces, peritoneal fluids, pleural effusion, sweat, tears, aqueous humor, pericardial fluid, lymph, chyme, chyle, bile, stool water, amniotic fluid, breast milk, pancreatic juice, cerumen, Cowper's fluid or pre-ejaculatory fluid, female ejaculate, interstitial fluid, menses, mucus, pus, sebum, vaginal lubrication, vomit |
| *Neurodegenerative*/*neurological disorders:* Parkinson's disease, Alzheimer's Disease and multiple sclerosis, Schizophrenia, and bipolar disorder, spasticity disorders, epilepsy | Blood, serum, plasma, CSF, urine |
| *Cardiovascular Disease:* atherosclerosis, cardiomyopathy, endocarditis, vunerable plaques, infection | Blood, serum, plasma, CSF, urine |
| *Stroke:* ischemic, intracerebral hemorrhage, subarachnoid hemorrhage, transient ischemic attacks (TIA) | Blood, serum, plasma, CSF, urine |
| *Pain disorders:* peripheral neuropathic pain and chronic neuropathic pain, and fibromyalgia, | Blood, serum, plasma, CSF, urine |
| *Autoimmune disease:* systemic and localized diseases, rheumatic disease, Lupus, Sjogren's syndrome | Blood, serum, plasma, CSF, urine, synovial fluid |
| *Digestive system abnormalities:* Barrett's esophagus, irritable bowel syndrome, ulcerative colitis, Crohn's disease, Diverticulosis and Diverticulitis, Celiac Disease | Blood, serum, plasma, CSF, urine |
| *Endocrine disorders*: diabetes mellitus, various forms of Thyroiditis, adrenal disorders, pituitary disorders | Blood, serum, plasma, CSF, urine |
| *Diseases and disorders of the skin:* psoriasis | Blood, serum, plasma, CSF, urine, synovial fluid, tears |
| *Urological disorders:* benign prostatic hypertrophy (BPH), polycystic kidney disease, interstitial cystitis | Blood, serum, plasma, urine |
| *Hepatic disease*/*injury:* Cirrhosis, induced hepatotoxicity (due to exposure to natural or synthetic chemical sources) | Blood, serum, plasma, urine |
| *Kidney disease*/*injury:* acute, sub-acute, chronic conditions, Podocyte injury, focal segmental glomerulosclerosis | Blood, serum, plasma, urine |
| Endometriosis | Blood, serum, plasma, urine, vaginal fluids |
| Osteoporosis | Blood, serum, plasma, urine, synovial fluid |
| Pancreatitis | Blood, serum, plasma, urine, pancreatic juice |
| Asthma | Blood, serum, plasma, urine, sputum, bronchiolar lavage fluid |
| Allergies | Blood, serum, plasma, urine, sputum, bronchiolar lavage fluid |
| Prion-related diseases | Blood, serum, plasma, CSF, urine |
| *Viral Infections:* HIV/AIDS | Blood, serum, plasma, urine |
| Sepsis | Blood, serum, plasma, urine, tears, nasal lavage |
| Organ rejection/transplantation | Blood, serum, plasma, urine, various lavage fluids |
| *Differentiating conditions:* adenoma versus hyperplastic polyp, irritable bowel syndrome (IBS) versus normal, classifying Dukes stages A, B, C, and/or D of colon cancer, adenoma with low-grade hyperplasia versus high-grade hyperplasia, adenoma versus normal, colorectal cancer versus normal, IBS versus, ulcerative colitis (UC) versus Crohn's disease (CD), | Blood, serum, plasma, urine, sputum, feces, colonic lavage fluid |
| *Pregnancy related physiological states, conditions, or affiliated diseases:* genetic risk, adverse pregnancy outcomes | Maternal serum, plasma, amniotic fluid, cord blood |

The biomarker profile obtained using one or more click-modified aptamer can be derived from a blood sample or blood derivative. Blood derivatives comprise plasma and serum. Blood plasma is the liquid component of whole blood, and makes up approximately 55% of the total blood volume. It is composed primarily of water with small amounts of minerals, salts, ions, nutrients, and proteins in solution. In whole blood, red blood cells, leukocytes, and platelets are suspended within the plasma. Blood serum refers to blood plasma without fibrinogen or other clotting factors (i.e., whole blood minus both the cells and the clotting factors).

Furthermore, a biological sample can comprise a vesicle or cell membrane fragment that is derived from a cell of origin and available extracellularly in a biological fluid or extracellular milieu.

One or more click-modified aptamer can be used to detect a microvesicle. A microvesicle, as used herein, comprises membrane vesicles that are shed from cells. Microvesicles or membrane vesicles include without limitation: circulating microvesicles (cMVs), vesicle, exosome, nanovesicle, dexosome, bleb, blebby, prostasome, microparticle, intralumenal vesicle, membrane fragment, intralumenal endosomal vesicle, endosomal-like vesicle, exocytosis vehicle, endosome vesicle, endosomal vesicle, apoptotic body, multivesicular body, secretory vesicle, phospholipid vesicle, liposomal vesicle, argosome, texasome, secresome, tolerosome, melanosome, oncosome, or exocytosed vehicle. Furthermore, although microvesicles may be produced by different cellular processes, the methods of the invention are not limited to or reliant on any one mechanism, insofar as such microvesicles are present in a biological sample and are capable of being characterized by the methods disclosed herein. Unless otherwise specified, methods that make use of a species of microvesicle can be applied to other types of microvesicles. Microvesicles generally comprise spherical structures with a lipid bilayer similar to cell membranes which surrounds an inner lumen which can contain soluble components, sometimes referred to as the payload. In some embodiments, the methods of the present disclosure make use of exosomes, which are small secreted vesicles of about 40-100 nm in diameter. For a review of membrane vesicles, including types and characterizations, see Thery et al., Nat Rev Immunol. 2009 Aug;9(8):581-93*.* Some properties of different types of vesicles include those in **Table 2:**

**Table 2: Vesicle Properties**

| **Feature** | **Exosomes** | **Microvesicles** | **Ectosomes** | **Membrane particles** | **Exosome-like vesicles** | **Apoptotic vesicles** |
|---|---|---|---|---|---|---|
| Size | 50-100 nm | 100-1,000 nm | 50-200 nm | 50-80 nm | 20-50 nm | 50-500 nm |
| Density in sucrose | 1.13-1.19 g/ml | | | 1.04-1.07 g/ml | 1.1 g/ml | 1.16-1.28 g/ml |
| EM appearance | Cup shape | Irregular shape, electron dense | Bilamellar round structures | Round | Irregular shape | Heterogeneo us |
| Sedimentation | 100,000 g | 10,000 g | 160,000-200,000 g | 100,000-200,000 g | 175,000 g | 1,200 g, 10,000 g, 100,000 g |
| Lipid composition | Enriched in cholesterol, sphingomyelin and ceramide; contains lipid rafts; expose PPS | Expose PPS | Enriched in cholesterol and diacylglycerol; expose PPS | | No lipid rafts | |
| Major protein markers | Tetraspanins (e.g., CD63, CD9), Alix, TSG101 | Integrins, selectins and CD40 ligand | CR1 and proteolytic enzymes; no CD63 | CD133; no CD63 | TNFRI | Histones |
| Intracellular origin | Internal compartments (endosomes) | Plasma membrane | Plasma membrane | Plasma membrane | | |

| | | | | | | |
|---|---|---|---|---|---|---|
| *Abbreviations:* phosphatidylserine (PPS); electron microscopy (EM) | | | | | | |

Microvesicles include shed membrane bound particles, or "microparticles," that are derived from either the plasma membrane or an internal membrane. Microvesicles can be released into the extracellular environment from cells. Cells releasing microvesicles include without limitation cells that originate from, or are derived from, the ectoderm, endoderm, or mesoderm. The cells may have undergone genetic, environmental, and/or any other variations or alterations. For example, the cells can be tumor cells. A microvesicle can reflect any changes in the source cell, and thereby reflect changes in the originating cells, e.g., cells having various genetic mutations. In one mechanism, a microvesicle is generated intracellularly when a segment of the cell membrane spontaneously invaginates and is ultimately exocytosed (see for example, Keller et al., Immunol. Lett. 107 (2): 102-8 (2006*)*). Microvesicles also include cell-derived structures bounded by a lipid bilayer membrane arising from both herniated evagination (blebbing) separation and sealing of portions of the plasma membrane or from the export of any intracellular membrane-bounded vesicular structure containing various membrane-associated proteins of tumor origin, including surface-bound molecules derived from the host circulation that bind selectively to the tumor-derived proteins together with molecules contained in the microvesicle lumen, including but not limited to tumor-derived microRNAs or intracellular proteins. Blebs and blebbing are further described in Charras et al., Nature Reviews Molecular and Cell Biology, Vol. 9, No. 11, p. 730-736 (2008*).* A microvesicle shed into circulation or bodily fluids from tumor cells may be referred to as a "circulating tumor-derived microvesicle." When such microvesicle is an exosome, it may be referred to as a circulating-tumor derived exosome (CTE). In some instances, a microvesicle can be derived from a specific cell of origin. CTE, as with a cell-of-origin specific microvesicle, typically have one or more unique biomarkers that permit isolation of the CTE or cell-of-origin specific microvesicle, e.g., from a bodily fluid and sometimes in a specific manner. For example, a cell or tissue specific markers are used to identify the cell of origin. Examples of such cell or tissue specific markers are disclosed herein and can further be accessed in the Tissue-specific Gene Expression and Regulation (TiGER) Database, available at bioinfo.wilmer.jhu.edu/tiger/; Liu et al. (2008) TiGER: a database for tissue-specific gene expression and regulation. BMC Bioinformatics. 9:271; TissueDistributionDBs, available at genome.dkfz-heidelberg.de/menu/tissue_db/index.html.

A microvesicle can have a diameter of greater than about 10 nm, 20 nm, or 30 nm. A microvesicle can have a diameter of greater than 40 nm, 50 nm, 100 nm, 200 nm, 500 nm, 1000 nm, 1500 nm, 2000 nm or greater than 10,000 nm. A microvesicle can have a diameter of about 20-2000 nm, about 20-1500 nm, about 30-1000 nm, about 30-800 nm, about 30-200 nm, or about 30-100 nm. In some embodiments, the microvesicle has a diameter of less than 10,000 nm, 2000 nm, 1500 nm, 1000 nm, 800 nm, 500 nm, 200 nm, 100 nm, 50 nm, 40 nm, 30 nm, 20 nm or less than 10 nm. As used herein the term "about" in reference to a numerical value means that variations of 10% above or below the numerical value are within the range ascribed to the specified value. Typical sizes for various types of microvesicles are shown in **Table 2.** Microvesicles can be assessed to measure the diameter of a single microvesicle or any number of microvesicles. For example, the range of diameters of a microvesicle population or an average diameter of a microvesicle population can be determined. Microvesicle diameter can be assessed using methods known in the art, e.g., imaging technologies such as electron microscopy. In an embodiment, a diameter of one or more microvesicles is determined using optical particle detection. See, e.g., U.S. Patent 7,751,053, entitled "Optical Detection and Analysis of Particles" and issued July 6, 2010; and U.S. Patent 7,399,600, entitled "Optical Detection and Analysis of Particles" and issued July 15, 2010.

In some embodiments, the methods of the present disclosure comprise assessing microvesicles directly such as in a biological sample without prior isolation, purification, or concentration from the biological sample. For example, a blood sample may be contacted with a click-modified aptamer in order to capture and/or detect (e.g., label) microvesicles in the sample. For example, the amount of microvesicles in the sample can by itself provide a biomarker profile that provides a diagnostic, prognostic or theranostic determination. Alternatively, the microvesicle in the sample may be isolated, captured, purified, or concentrated from a sample prior to analysis. As noted, isolation, capture or purification as used herein comprises partial isolation, partial capture or partial purification apart from other components in the sample. Microvesicle isolation can be performed using various techniques known in the art, e.g., chromatography, filtration, centrifugation, flow cytometry, affinity capture (e.g., to a planar surface or bead), and/or using microfluidics. See e.g., International Patent Publications WO/2010/056337, WO 2011/109440 WO 2011/127219, WO 2012/024543, WO 2009/015357, WO 2011/088226, WO 2012/0115885, WO 2012/174282, WO 2013/022995, WO 2012/170711 for further details of microvesicle assessment.

Microvesicles can be assessed to provide a phenotypic characterization by comparing microvesicle characteristics to a reference. In some embodiments, surface antigens on a microvesicle are assessed. The surface antigens can provide an indication of the anatomical origin and/or cellular of the microvesicles and other phenotypic information, e.g., tumor status. Microvesicles are found in a patient sample, e.g., a bodily fluid such as blood, serum or plasma, can be assessed for surface antigens indicative of origin and the presence of a cancer. The surface antigens may comprise any informative biological entity that can be detected on the microvesicle membrane surface, including without limitation surface proteins, lipids, carbohydrates, and other membrane components. As a non-limiting example, positive detection of colon derived microvesicles expressing tumor antigens can indicate that the patient has colorectal cancer. As such, methods of the present disclosure can be used to characterize any disease or condition associated with an anatomical or cellular origin, by assessing, for example, disease-specific and cell-specific biomarkers of one or more microvesicles obtained from a subject. The click-modified aptamers can be used to capture and/or detect (e.g., label) microvesicles as desired.

The methods may comprise assessing one or more microvesicle payload to provide a phenotypic characterization. The payload within a microvesicle comprises any informative biological entity that can be detected as encapsulated within the microvesicle, including without limitation proteins and nucleic acids, e.g., genomic or cDNA, mRNA, or functional fragments thereof, as well as microRNAs (miRs). In addition, methods of the present disclosure are directed to detecting microvesicle surface antigens (in addition or exclusive to microvesicle payload) to provide a phenotypic characterization. For example, microvesicles can be characterized by using binding agents (e.g., click-modified aptamers) that are specific to microvesicle surface antigens, and the bound microvesicles can be further assessed to identify one or more payload components disclosed therein. A click-modified aptamer may be used to assess payload as well, e.g., in an immunoassay format. As described herein, the levels of microvesicles with surface antigens of interest or with payload of interest can be compared to a reference to characterize a phenotype. For example, overexpression in a sample of cancer-related surface antigens or microvesicle payload, e.g., a tumor associated mRNA or microRNA, as compared to a reference, can indicate the presence of cancer in the sample. The biomarkers assessed can be present or absent, increased or reduced based on the selection of the desired target sample and comparison of the target sample to the desired reference sample. Non-limiting examples of target samples include: disease; treated/not-treated; different time points, such as a in a longitudinal study; and non-limiting examples of reference sample: non-disease; normal; different time points; and sensitive or resistant to candidate treatment(s).

### Therapeutics

As used herein "therapeutically effective amount" refers to an amount of a composition that relieves (to some extent, as judged by a skilled medical practitioner) one or more symptoms of the disease or condition in a mammal. Additionally, by "therapeutically effective amount" of a composition is meant an amount that returns to normal, either partially or completely, physiological or biochemical parameters associated with or causative of a disease or condition. A clinician skilled in the art can determine the therapeutically effective amount of a composition in order to treat or prevent a particular disease condition, or disorder when it is administered, such as intravenously, subcutaneously, intraperitoneally, orally, or through inhalation. The precise amount of the composition required to be therapeutically effective will depend upon numerous factors, e.g., such as the specific activity of the active agent, the delivery device employed, physical characteristics of the agent, purpose for the administration, in addition to many patient specific considerations. But a determination of a therapeutically effective amount is within the skill of an ordinarily skilled clinician upon the appreciation of the disclosure set forth herein.

The terms "treating," "treatment," "therapy," and "therapeutic treatment" as used herein refer to curative therapy, prophylactic therapy, or preventative therapy. An example of "preventative therapy" is the prevention or lessening the chance of a targeted disease (e.g., cancer or other proliferative disease) or related condition thereto. Those in need of treatment include those already with the disease or condition as well as those prone to have the disease or condition to be prevented. The terms "treating," "treatment," "therapy," and "therapeutic treatment" as used herein also describe the management and care of a mammal for the purpose of combating a disease, or related condition, and includes the administration of a composition to alleviate the symptoms, side effects, or other complications of the disease, condition. Therapeutic treatment for cancer includes, but is not limited to, surgery, chemotherapy, radiation therapy, gene therapy, and immunotherapy.

As used herein, the term "agent" or "drug" or "therapeutic agent" refers to a chemical compound, a mixture of chemical compounds, a biological macromolecule, or an extract made from biological materials such as bacteria, plants, fungi, or animal (particularly mammalian) cells or tissues that are suspected of having therapeutic properties. The agent or drug can be purified, substantially purified or partially purified. An "agent" also includes a radiation therapy agent or a "chemotherapuetic agent."

As used herein, the term "diagnostic agent" refers to any chemical used in the imaging of diseased tissue, such as, e.g., a tumor.

As used herein, the term "chemotherapuetic agent" refers to an agent with activity against cancer, neoplastic, and/or proliferative diseases, or that has ability to kill cancerous cells directly.

As used herein, "pharmaceutical formulations" include formulations for human and veterinary use with no significant adverse toxicological effect. "Pharmaceutically acceptable formulation" as used herein refers to a composition or formulation that allows for the effective distribution of the nucleic acid molecules in the physical location most suitable for their desired activity.

As used herein the term "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated.

The click-modifed aptamers can be used directly for therapeutic applications, e.g., to block binding of target of interest. The click-modifed aptamers can be conjugated to toxins or other effector molecules. Extensive previous work has developed the concept of antibody-toxin conjugates ("immunoconjugates") as potential therapies for a range of indications, e.g., directed at the treatment of cancer. A variety of different therapeutic agents for targeted delivery have been tested in pre-clinical and clinical studies, including protein toxins, high potency small molecule cytotoxics, radioisotopes, and liposome-encapsulated drugs. Although these efforts have successfully yielded FDA-approved therapies for hematological tumors, immunoconjugates as a class (especially for solid tumors) have historically yielded disappointing results that have been attributable to multiple different properties of antibodies, including tendencies to develop neutralizing antibody responses to non-humanized antibodies, limited penetration in solid tumors, loss of target binding affinity as a result of toxin conjugation, and imbalances between antibody half-life and toxin conjugate half-life that limit the overall therapeutic index (reviewed by Reff and Heard, Critical Reviews in Oncology/Hematology, 40 (2001):25-35).

Aptamers are functionally similar to antibodies, except their absorption, distribution, metabolism, and excretion ("ADME") properties are intrinsically different and they generally lack many of the immune effector functions generally associated with antibodies (e.g., antibody-dependent cellular cytotoxicity, complement-dependent cytotoxicity). In comparing many of the properties of aptamers and antibodies previously described, several factors suggest that toxin-delivery via aptamers offers several concrete advantages over delivery with antibodies, ultimately affording them better potential as therapeutics. Several examples of the advantages of toxin-delivery via aptamers over antibodies are as follows:
1) Aptamer-toxin conjugates are entirely chemically synthesized. Chemical synthesis provides more control over the nature of the conjugate. For example, the stoichiometry (ratio of toxins per aptamer) and site of attachment can be precisely defined. Different linker chemistries can be readily tested. The reversibility of aptamer folding means that loss of activity during conjugation is unlikely and provides more flexibility in adjusting conjugation conditions to maximize yields.
2) Smaller size allows better tumor penetration. Poor penetration of antibodies into solid tumors is often cited as a factor limiting the efficacy of conjugate approaches. See Colcher, D., Goel, A., Pavlinkova, G., Beresford, G., Booth, B., Batra, S. K. (1999) "Effects of genetic engineering on the pharmacokinetics of antibodies," Q. J. Nucl. Med., 43: 132-139. Studies comparing the properties of unPEGylated anti-tenascin C aptamers with corresponding antibodies demonstrate efficient uptake into tumors (as defined by the tumor:blood ratio) and evidence that aptamer localized to the tumor is unexpectedly long-lived (t_{1/2}>12 hours) (Hicke, B. J., Stephens, A. W., "Escort aptamers: a delivery service for diagnosis and therapy", J. Clin. Invest., 106:923-928 (2000)).
3) Tunable PK. Aptamer half-life/metabolism can be easily tuned to match properties of payload, optimizing the ability to deliver toxin to the tumor while minimizing systemic exposure. Appropriate modifications to the aptamer backbone and addition of high molecular weight PEGs should make it possible to match the half-life of the aptamer to the intrinsic half-life of the conjugated toxin/linker, minimizing systemic exposure to non-functional toxin-bearing metabolites (expected if t_{1/2}(aptamer)<<t_{1/2}(toxin)) and reducing the likelihood that persisting unconjugated aptamer will functionally block uptake of conjugated aptamer (expected if t_{1/2}(aptamer)>>t_{1/2} (toxin)).
4) Relatively low material requirements. It is likely that dosing levels will be limited by toxicity intrinsic to the cytotoxic payload. As such, a single course of treatment will likely entail relatively small (<100 mg) quantities of aptamer, reducing the likelihood that the cost of oligonucleotide synthesis will be a barrier for aptamer-based therapies.
5) Parenteral administration is preferred for this indication. There will be no special need to develop alternative formulations to drive patient/physician acceptance.

The present disclosure provides a pharmaceutical composition comprising a therapeutically effective amount of a click-modified aptamer provided by the invention or a salt thereof, and a pharmaceutically acceptable carrier or diluent. The invention also provides a pharmaceutical composition comprising a therapeutically effective amount of the aptamer or a salt thereof, and a pharmaceutically acceptable carrier or diluent. Relatedly, the present disclosure provides a method of treating or ameliorating a disease or disorder, comprising administering the pharmaceutical composition to a subject in need thereof. Administering a therapeutically effective amount of the composition to the subject may result in: (a) an enhancement of the delivery of the active agent to a disease site relative to delivery of the active agent alone; or (b) an enhancement of target clearance resulting in a decrease of at least 10%, 20%, 30%, 40%, 50%, 60%, 70%, 80%, or 90% in a blood level of the target of the aptamer, e.g., a circulating protein or microvesicle; or (c) an decrease in biological activity of the target of the aptamer, e.g., a circulating protein or microvesicle,of at least 10%, 20%, 30%, 40%, 50%, 60%, 70%, 80%, or 90%. In an embodiment, the biological activity of microvesicles comprises immune suppression or transfer of genetic information. The disease or disorder can include without limitation those disclosed herein. For example, the disease or disorder may comprise a neoplastic, proliferative, or inflammatory, metabolic, cardiovascular, or neurological disease or disorder. See, e.g., section "Phenotypes" herein.

### EXAMPLES

### Material

**Nucleic Acids:**

| name | specification | supplier |
|---|---|---|
| Library (Cy5-FT2-N42-EdU): Cy5-CAC GAC GCA AGG GAC CAC AGG NNN NNN NNN NNN NNN NNN NNN NNN NNN NNN NNN NNN NNN NNN CAG CAC GAC ACC GCA GAG GCA; (SEQ ID NO: 1) | | Ella Biotech, Germany |
| N: dA, dC, dG, Ethynyl-dU (1:1:1:1) CAC GAC GCA AGG GAC CAC AGG (SEQ ID NO: 2) | forward primer (FT2-F) | Ella Biotech, Germany |
| Cy5-CAC GAC GCA AGG GAC CAC AGG (SEQ ID NO: 2) | forward primer (FT2-F-Cy5) | Microsynth AG, Switzerland |
| Phosphate-TGC CTC TGC GGT GTC GTG CTG (SEQ ID NO: 3) | reverse primer (FT2-R-P) | Microsynth AG, Switzerland |

**Proteins:**

| name | specification | supplier | Catalogue no. |
|---|---|---|---|
| *Pwo* DNA polymerase | 2.5 U / µl | Genaxxon, Germany | M3002.0100 |
| λ-Exonuclease | 10 U / µl | Thermo Scientific | EN0562 |
| Bovine Serum Albumine (BSA) | | Sigma-Aldrich | A9418-10G |
| T4 Polynucleotide Kinase | | New England Biolabs | M0201L |
| Green Fluorescent Protein (GFP | | Life Technologies | 13105-S07E-250 |

**Chemicals:**

| name | specification | supplier | Catalogue no. |
|---|---|---|---|
| Tris(4-(3-hydroxy-propoyl)-[1,2,3]triazol-1-ylmethyl)amine (THPTA) | | Baseclick, Germany | BCMI-006-5 |
| Copper sulphate | | Sigma-Aldrich | 451657-10G |
| 5-Ethynyl-5'-*O*-triphosphate-2'-deoxyuridine (EdUTP) | | Baseclick, Germany | BCT-08-L |
| dNTPs | | Larova, Germany | 250 µM |
| Dulbecco's Phosphate Buffered Saline (D-PBS) | | Sigma-Aldrich | D8662-1L |
| Salmon Sperm DNA | | Life Technologies | AM9680 |
| TWEEN® 20 | | Merck Millipore, Germany | 8170721000 |
| ATP[γ-³²P]-3000Ci/mmol 10mCi/ml | | Perkin Elmer | BLU002A500UC |

**Miscellaneous**

| name | supplier | Catalogue no. |
|---|---|---|
| Dynabeads® His-Tag Isolation & Pulldown | Life Technologies | 10103D |
| DynaMag™-2 magnetic rack | Life Technologies | 12321D |
| NucleoSpin® Gel and PCR Clean-up | Macherey-Nagel, Germany | 740609.250 |

**Buffers:**

| name | specification |
|---|---|
| SELEX-Buffer | 1x D-PBS + 0.1 % BSA + 0.1 mg/ml salmon sperm + 0.01% Tween20 |
| B15-Buffer | 25 mM Tris, 30 mM NaCl, 1 mM KCl, 1 mM CaCl₂, 1 mM MgCl₂; pH 8.3 |

### Example 1

### Synthesis of 3-(2-Azidoethyl)-1-indole

Under an argon-atmosphere, 0.90 g 3-(2-Bromoethyl)-1H-indole (4.02 mmol) was solved in 10 mL dry DMF and 0.26 g sodium azide (4.02 mmol) was added. After 15 hours at 25 °C the solution was washed sequentially with water. The combined aqueous layers where reextracted with ethylacetate and the combined ethylacetate solutions were dried with MgSO₄ and concentrated. Column chromatography (ethylacetate/petroleum ether 1:9) gave 3-(2-Azidoethyl)-1H-indole as yellow oil. The yield was 0.52 g (70%).

### Example 2

The method targeting the Green Fluorescent Protein (GFP) and employing an Indole modification

### 2.1 - Preparing a candidate mixture of nucleic acids

The single stranded DNA starting library comprising 42 wooble positions (Cy5-FT2-N42-EdU): Cy5-CAC GAC GCA AGG GAC CAC AGG NNN NNN NNN NNN NNN NNN NNN NNN NNN NNN NNN NNN NNN NNN CAG CAC GAC ACC GCA GAG GCA; N: dA, dC, dG, Ethynyl-dU (1:1:1:1)) (SEQ ID NO: 1) was synthesized and HPLC purified by Ella Biotech (Munich, Germany).

The library was further functionalized with the Indole-Azide prepared according to Example 1 in a Cu(I) catalysed azide-alkyne cycloaddition (CuAAC reaction) as follows: 150 µl of a 3.5 µM DNA solution were mixed with 20 µl D-PBS and 20 µl 10 mM azide solution and the reaction was started by addition of 10 µl freshly prepared catalyst solution (10 mM THPTA, 2 mM CuSO₄, 50 mM Sodium ascorbate). The reaction mixture was incubated for 1h at 37 °C, 800 rpm and the functionalized ssDNA was purified with the NucleoSpin® Gel and PCR Clean-up kit according to the manufacturers recommendation.

### 2.2 - Preparation of the target sample

50 µg recombinant expressed and purified His6-tagged GFP (His6-GFP) (Life Technologies) were immobilized on 5 mg magnetic beads (Dynabeads His-Tag Isolation) in a total volume of 500 µl SELEX buffer (1x D-PBS + 0.1 % BSA + 0.1 mg/ml salmon sperm + 0.01% Tween20), according to the manufacturers recommendation. The GFP loaded beads were stored at 4°C until further usage.

### 2.3 - Contacting the candidate mixture with the target sample and partitioning the nucleic acids that bind to GFP from those that do not bind

100 µl (ca. 500 pmol) clicked and purified DNA start library of step 5.1 in 1X D-PBS were heated to 95°C for 3 min and let slowly cool to ambient temperature afterwards. 50 µl (500 µg) uncoupled magnetic beads (in SELEX buffer) were added and incubated for 30 min at 37 °C, 800 rpm. The supernatant was added to 50 µl (500 µg) GFP-bead suspension and incubated for 30 min at 37 °C, 800 rpm. The supernatant was discarded and the beads were washed 3 times for 30 sec with 200 µl SELEX buffer. 100 µl 300 mM imidazole-solution were added and incubated for 15 min at 37°C, 800 rpm. This supernatant was used for the subsequent PCR amplification.

### 2.4 - Amplification of the binding nucleic acids

The eluted DNA (enriched library) was divided into 8 aliquots and PCR amplified as follows: All PCR reactions were done in 100 µl reaction volume according to the following pipetting and cycling scheme of table 1 in a Mastercycler® Personal (Eppendorf) thermocycler. The samples were cycled until the desired product band could be detected on an ethidium bromide stained 4% agarose gel.

**Table 5:**

| Reagent | Stock-Conc. | Final-Conc. |
|---|---|---|
| Water | | |
| DNA solution | | |
| PWO-Buffer | 10X | 1X |
| dN^{∗}TPs | 25 mM | 250 µM |
| F-Primer | 50 µM | 0.5 µM |
| R-Primer | 50 µM | 0.5 µM |
| PWO-Pol. | 2.5 U/µl | 2.5 U |

| | | |
|---|---|---|
| dN^{∗}TPs: dATP / dCTP / dGTP / Ethynyl-dUTP | | |

The cycling scheme was as follows: 2 min at 95°C, followed by 30 sec at 95 °C / 30 sec at 62 °C / 1 min at 72 °C for X cycles, storage at 4 °C.

The dsDNA PCR products were purified with the NucleoSpin® Gel and PCR Clean-up kit according to the manufacturers recommendation and as a backup of this SELEX round 10 % of the purified dsDNA were stored at -20 °C.

### 2.5 - Single strand displacement

Purified dsDNA PCR product was supplemented with 40 µl 10 X exonuclease reaction buffer and 8 µl lambda exonuclease were added. The mixture was incubate for 60 min at 37 °C, 800 rpm and the ssDNA digestion product was purified with the NucleoSpin® Gel and PCR Clean-up kit according to the manufacturers recommendation

### 2.6 - Functionalization of the enriched library

For the next SELEX round the enriched single stranded library was again further functionalized with the Indol-Azide in a CuAAC reaction as follows: 150 µl of the DNA solution were mixed with 20 µl D-PBS and 20 µl 10mM azide solution and the reaction was started by addition of 10 µl freshly prepared catalyst solution (10 mM THPTA, 2 mM CuSO4, 50 mM Sodium ascorbate). The reaction mixture was incubated for 1h at 37 °C, 800 rpm and the functionalized ssDNA was purified with the NucleoSpin® Gel and PCR Clean-up kit according to the manufacturers recommendation.

### 2.7 - 2^{nd} to 15^{th} SELEX round

10 µl 10 X D-PBS were added to 90 µl of the enriched clicked DNA library (ca. 50 pmol) and the next SELEX round was performed as described for the first round. In total 15 rounds of SELEX were performed. To increase the selection pressure the amount of GFP-functionalized beads was reduced and the washing times were increased as summarized in the following table 6:

**Table 6:**

| Round | GFP-beads [µl] | Wash 1 | Wash 2 | Wash 3 |
|---|---|---|---|---|
| 1 | 50 | 30 sec | 30 sec | 30 sec |
| 2 | 50 | 30 sec | 5 min | 30 sec |
| 3 | 50 | 30 sec | 5 min | 30 sec |
| 4 | 25 | 30 sec | 5 min | 30 sec |
| 5 | 10 | 30 sec | 5 min | 5 min |
| 6-15 | 5 | 30 sec | 5 min | 5 min |

### 2.8 - Fluorescence activated cell sorting (FACS) Binding Assay

45 µl of the respective Cy5 labelled single strand DNA solution and 5 µl of the respective protein immobilized bead-suspension were incubated for 30 min at 37 °C, 800 rpm. The supernatant was discarded and the beads were washed 1 time for 30 sec and 1 time for 5 min with 200 µl SELEX buffer. The beads were resuspended in 200 µl D-PBS and 100000 events were recorded in a FACScantoII (BD Biosciences) flow cytometer measuring the Cy5 fluorescence in the red channel.

The Figure 2 shows the results of a FACS binding assay between the 15^{th} round enriched library and GFP-coated magnetic beads. The Cy5-labelled, 15^{th} round library leads to a concentration dependent, much higher mean fluorescence intensity on GFP-beads compared to the starting library. This increase in click dependent binding affinity demonstrates the successful enrichment of GFP binding sequences after 15 rounds of click SELEX.

The Figure 3-A shows "C12" a monoclonal sequence isolated from the 15^{th} round enriched library, containing six ethynyl-dU (EdU) clickable nucleotides. Figure 3-B shows schematically the chemical structure of one ethynyl-dU nucleotide incorporated in a DNA strand.

The Figure 4 shows the results of a FACS binding assay between the "C12" monoclonal sequence depicted in figure 7-A and protein-coated magnetic beads. C12 leads to a concentration dependent, much higher mean fluorescence intensity on the GFP-beads compared to the starting library. However, on ERK2 or streptavidin coated magnetic beads the situation is reversed and C12 shows less mean fluorescence intensity than the start library. This shows C12s high specificity to GFP compared to the two control proteins.

### Example 3

### Biomarker Detection

This Example illustrates the use of the click-modified aptamers identified by the method of the present invention to determine a biomarker profile in a biological sample.

### 3.1 - Biomarker capture

A click-modified aptamer is identified using the methods of the invention that binds to protein target of interest. The aptamer is attached directly or indirectly a surface such as a planar array, well, microbead or column matrix. A linker may be used to provide a desired spacing between the aptamer and the surface. The biological sample is contacted with the surface and the aptamer is allowed to capture any target that is present in the sample.

### 3.2 - Biomarker detection

A click-modified aptamer is identified using the methods of the invention that binds to protein target of interest. The aptamer is labelled, e.g., with a fluorescent, radioactive, enzymatic or metal label. The aptamer is contacted with a sample comprising or suspected to comprise the target of the aptamer. The label is used to detect a complex formed between the aptamer and its target.

### 3.3 - Formats

One of skill will appreciate that one of more aptamer can be used in a variety of assays in which antibodies are traditionally used. Such assays include without limitation immunoassays, ELISA, Western blot, sandwich assays, immunoprecipitation, affinity column, flow cytometry, microbead assays, and proteomic arrays. If available and desireable, aptamers can be used for both capture and detection in a single assay, e.g., as both components in a sandwich assay format.

In another exemplary format, one or more aptamer identified by the methods of the invention are contacted with the sample, the aptamers that bind the sample are collected, and the aptamers are sequenced in order to identify aptamers that bind the sample. The sequencing can be performed using traditional or Next Generation sequencing technology.

### Example 4

### Disease Diagnosis

This Example illustrates the use of the click-modified aptamers identified by the method of the present invention to diagnose a proliferative disease.

A suitable quantity of an aptamer that binds a cancer-derived microvesicle is synthesized via chemical means known in the art. The aptamers are conjugated to a diagnostic agent suitable for detection, such as a fluorescent moiety, using a conjugation method known in the art.

The composition is applied to microvesicles isolated from blood samples taken from a test cohort of patients suffering from a proliferative disease associated with the overexpression of microvesicles, e.g., prostate, breast, or lung cancer. The composition is likewise applied to microvesicles isolated from blood samples taken from a negative control cohort, not suffering from a proliferative disease.

The use of appropriate detection techniques (e.g., microbead assay or flow cytometry) on the test cohort samples indicates the presence of disease, while the same techniques applied to the control cohort samples indicate the absence of disease.

The results show that the aptamers are useful in diagnosing proliferative diseases.

### Example 5

### Treatment Selection and Monitoring for Prostate Cancer

This Example uses an aptamer for detecting a biomarker profile for prostate cancer. The click-modified aptamer is identified by selecting aptamers that bind prostate cancer derived microvesicles. The microvesicles are detected in a blood sample from a patient by using an aptamer to capture the microvesicles. The amount of detected microvesicles is compared to a reference value indicative of a healthy state to provide the biomarker profile.

The biomarker profile is used to monitor an efficacy of a treatment for the prostate cancer. A patient is identified with a suspicious serum PSA level (e.g., serum PSA > 4.0 ng/ml) and/or a suspicious digital rectal examination (DRE). The biomarker profile is determined for the patient, and the results are found to be positive for prostate cancer. The treating physician determines whether to treat the prostate cancer with a therapeutic agent, hormone therapy, and/or a surgery (prostatectomy). After treatment, the biomarker profile is again determined for the patient. A positive result indicates a negative patient response to the treatment and that further treatment is required. A negative result indicates a positive patient response to the treatment and that further treatment may not be necessary.

### Example 6

### Therapeutic Aptamers

This Example illustrates the use a click-modified aptamer identified using the methods of the invention to treat a proliferative disease in a mouse.

A suitable quantity of an aptamer that binds a cancer-derived microvesicle is synthesized via chemical means known in the art. The aptamers are conjugated to a chemotherapeutic agent using a conjugation method known in the art. The conjugate is formulated in an aqueous composition.

The composition is administered intravenously, in one or more doses, to a test cohort of mice suffering from a proliferative disease associated with the overexpression of the microvesicles, e.g., prostate, breast, or lung cancer model. A control cohort, not suffering from a proliferative disease is administered the identical composition intravenously, according to a corresponding dosage regimen.

Pathological analysis of tumor samples and/or mouse survival indicate that mortality and/or morbidity are improved in the test cohort over the control cohort.

The results show that the aptamers are useful in treating proliferative diseases.

Aptamers determined to be useful in the mouse model are tested for treatment of the cancer in other organisms, e.g., a human.

## Claims

1. A method of identifying or producing an aptamer in an in vitro selection process comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the nucleobase that has been modified to contain the azide-alkyne group was an ethynyl-dU, dA, dC or dG nucleotide;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer,
wherein the binding nucleic acids are amplified by PCR using an ethynyl-modified nucleobase and again are subjected to an in vitro selection cycle until an enrichment of target-binding nucleic acids is detected.

2. The method of claim 1 further comprising displacing one strand of at least one nucleic acid amplified in step (d), and further modifying the nucleic acid to introduce another functionalization.

3. The method of claim 1, wherein the azide-alkyne modified nucleobase comprises aliphatic-, aromatic-, charged-, basic-, acidic, heteroaromatic-, sugar-kind of-, metal-containing- or peptide- residues.

4. The method of claim 1, wherein the amplification step (d) employs a polymerase chain reaction using an alkyne-modified nucleobase that is further modified via a 1,3 dipolar cycloaddition of an azide to yield an azide-alkyne modified nucleobase.

5. The method of claim 1, wherein the azide comprises a light-sensitive molecule.

6. The method of claim 1, wherein the target sample is selected from the group consisting of a polypeptide, peptide, cell, cell fragment, cell membrane, small organic molecule, cell culture, tissue sample, microvesicle, biological fluid sample and combination thereof.

## Patentansprüche

1. Verfahren zum Identifizieren oder Herstellen eines Aptamers in einem in vitro Selektionsverfahren umfassend die Schritte des:
(a) Herstellens einer Kandidatenmischung von Nucleinsäuren, wobei die Mischung mindestens ein Nucleotid umfasst, das modifiziert worden ist, um eine durch Click-Chemie eingeführte Funktionalisierung zu umfassen, wobei das modifizierte Nucleotid eine durch Alkyn modifizierte Nucleobase umfasst, die noch weiter über eine dipolare 1,3-Cycloaddition eines Azids modifiziert ist, um eine durch Azid-Alkyn modifizierte Nucleobase zu ergeben, wobei die Nucleobase, die modifiziert worden ist um eine Azid-Alkyn-Gruppe enthalten, ein Ethinyl-dU, dA, dC or dG Nucleotid war;
(b) Kontaktierens der Kandidatenmischung mit einer Targetprobe;
(c) Segmentierens von Nucleinsäuren, die sich an die Targetprobe binden, von denjenigen, die sich nicht binden;
(d) Amplifizierens der sich bindenden Nucleinsäuren, um eine Population von Nucleinsäuren herzustellen, die die Targetprobe binden, wodurch ein Aptamer identifiziert oder hergestellt wird,
wobei die sich bindenden Nucleinsäuren mittels PCR unter Verwendung einer Ethinyl-modifizierten Nucleobase amplifiziert werden, und erneut einem in vitro Selektionszyklus unterworfen werden bis eine Anreicherung von die Targetprobe bindenden Nucleinsäuren detektiert wird.

2. Verfahren nach Anspruch 1, umfassend ferner das Verdrängen eines Strangs mindestens einer Nucleinsäure, die in Schritt (d) amplifiziert worden ist, und das Modifizieren der Nucleinsäure, um eine andere Funktionalisierung einzuführen.

3. Verfahren nach Anspruch 1, wobei die durch Azid-Alkyn modifizierte Nucleobase aliphatische, aromatische, geladene, basische, saure, heteroaromatische, zuckerartige, metall-enthaltende oder Peptid-Reste umfasst.

4. Verfahren nach Anspruch 1, wobei im Amplifikationsschritt (d) eine Polymerasekettenreaktion unter Verwendung einer durch Alkyn modifizierten Nucleobase angewendet wird, die noch weiter über eine dipolare 1,3-Cycloaddition eines Azids modifiziert ist, um eine durch Azid-Alkyn modifizierte Nucleobase zu ergeben.

5. Verfahren nach Anspruch 1, wobei das Azid ein lichtempfindliches Molekül umfasst.

6. Verfahren nach Anspruch 1, wobei die Targetprobe aus der Gruppe ausgewählt ist bestehend aus einem Polypeptid, Peptid, einer Zelle, einem Zellfragment, einer Zellmembran, einem kleinen organischen Molekül, einer biologischen Fluidprobe und einer Kombination davon.

## Revendications

1. Méthode d'identification ou de production d'un aptamère dans un procédé de sélection *in vitro,* comprenant les étapes consistant à :
(a) préparer un mélange candidat d'acides nucléiques, où le mélange comprend au moins un nucléotide qui est modifié afin de comprendre une fonctionnalisation introduite par chimie « clic », où le nucléotide modifié comprend une nucléobase à modification alcyne qui est en outre modifiée via une cycloaddition 1,3-dipolaire d'un azoture afin de conduire à une nucléobase à modification azoture-alcyne, où la nucléobase ayant été modifiée afin de contenir le groupement azoture-alcyne était un nucléotide éthynyl-dU, dA, dC ou dG ;
(b) mettre en contact le mélange candidat avec un échantillon cible ;
(c) partager les acides nucléiques qui se fixent à l'échantillon cible de ceux qui ne se fixent pas ;
(d) amplifier les acides nucléiques se fixant, afin de produire une population d'acides nucléiques qui se fixent à l'échantillon cible, afin d'identifier ou de produire ainsi un aptamère ;
où les acides nucléiques se fixant sont amplifiés par PCR en utilisant une nucléobase à modification éthynyle et sont de nouveau soumis à un cycle de sélection *in vitro* jusqu'à ce qu'on détecte un enrichissement en acides nucléiques se fixant à la cible.

2. Méthode selon la revendication 1, comprenant en outre le déplacement d'un brin d'au moins un acide nucléique amplifié dans l'étape (d), et en outre la modification de l'acide nucléique afin d'introduire une autre fonctionnalisation.

3. Méthode selon la revendication 1, dans laquelle la nucléobase à modification azoture-alcyne comprend des restes aliphatiques, aromatiques, chargés, basiques, acides, hétéroaromatiques, de type sucre, contenant des métaux ou peptidiques.

4. Méthode selon la revendication 1, dans laquelle l'étape d'amplification (d) emploie une amplification en chaîne par polymérase en utilisant une nucléobase à modification alcyne qui est en outre modifiée via une cycloaddition 1,3-dipolaire d'un azoture afin de conduire à une nucléobase à modification azoture-alcyne.

5. Méthode selon la revendication 1, dans laquelle l'azoture comprend une molécule photosensible.

6. Méthode selon la revendication 1, dans laquelle l'échantillon cible est choisi dans le groupe constitué par un polypeptide, un peptide, une cellule, un fragment cellulaire, une membrane cellulaire, une petite molécule organique, une culture cellulaire, un échantillon tissulaire, une microvésicule, un échantillon de fluide biologique, et une combinaison de ceux-ci.


### SEQUENCE LISTING

<110> Mayer, Günter Tolle, Fabian
<120> A method of identifying or producing an aptamer
<130> UD 40748
<150> US 62/058703
   <151> 2014-10-02
<160> 3
<170> PatentIn version 3.3
<210> 1
   <211> 84
   <212> DNA
   <213> Artificial
<220>
   <223> library
<220>
   <221> misc_feature
   <222> (22)..(63)
   <223> n is a, c, g or u
<400> 1
   cacgacgcaa gggaccacag gnnnnnnnnn nnnnnnnnnn nnnnnnnnnn nnnnnnnnnn      60
   nnncagcacg acaccgcaga ggca                                              84
<210> 2
   <211> 21
   <212> DNA
   <213> Artificial
<220>
   <223> forward primer
<400> 2
   cacgacgcaa gggaccacag g 21
<210> 3
   <211> 21
   <212> DNA
   <213> Artificial
<220>
   <223> reverse primer
<400> 3
   tgcctctgcg gtgtcgtgct g 21